# EUROPEAN PATENT APPLICATION

(11) **EP 4 711 458 A2**
(43) Date of publication of application: **18.03.2026**
(21) Application number: 25180249.2
(22) Date of filing: 07.02.2022
(51) Int. Cl.: C12N 15/113

(54) **SELF-REPLICATING RNA AND USES THEREOF**

(62) Divisional of application: 22920982.0
(71) Applicant: Seqirus Inc., Holly Springs, NC 27540 (US)
(72) Inventor: ALLEN, Pirada, New York 27540 (US); DE SOUZA, Ivna, New York 27540 (US); WEN, Yingxia, New York 27540 (US); CHANG, Cheng, New York 27540 (US); LEE, Changkeun, New York 27540 (US)
(74) Representative: Finnegan Europe LLP

(57) **Abstract**

The present disclosure relates to self-replicating RNA encoding an antigen from severe acute respiratory syndrome coronavirus 2 (SARS-CoV-2) and uses thereof.

## Description

### SEQUENCE LISTING

The present application is filed together with a Sequence Listing in electronic form. The entire contents of the Sequence Listing are hereby incorporated by reference.

### FIELD

The present disclosure relates to self-replicating RNA encoding an antigen from severe acute respiratory syndrome coronavirus 2 (SARS-CoV-2) and uses thereof.

### BACKGROUND

In late 2019, a novel severe acute respiratory syndrome coronavirus (SARS-CoV-2) was identified in China in humans and is responsible for causing the severely infectious coronavirus disease 2019 (COVID-19). Since December 2019, the global spread of this virus has been widespread to all countries with the World Health Organisation declaring the outbreak a pandemic on March 11 2020. Human infection with this virus displays a broad clinical spectrum with a high rate of transmissibility. The global count of infections and mortality continues to rise with infections exceeding 54 million, recovery 35 million and over 1.3 million deaths to date.

Vaccines are the critical health intervention to prevent this infectious disease. This pandemic has seen the unprecedented development of multiple vaccines, with over 200 vaccines in development to date, over 30 in clinical trial and multiple in Phase 3. The majority of the vaccines that have been developed attempt to evoke the immune system to recognise the SARS-COV-2 spike protein (or S protein) since early studies of recombinant SARS-CoV protein in a hamster challenge model demonstrated that this approach was immunogenic and protective.

However, there are currently no approved therapeutic and/or prophylactic vaccines against SARS-CoV-2. Thus, there remains a need to develop specific and efficient vaccines against SARS-CoV-2. It is also desirable that such vaccines can be produced in sufficient amounts, particularly during a pandemic, and more rapidly than current egg-based techniques employed for manufacturing influenza vaccines.

### SUMMARY

The present disclosure is based on the inventors' identification of a self-replicating RNA against a severe acute respiratory syndrome coronavirus 2 (SARS-CoV-2) antigen.

The findings by the inventors provide the basis for a self-replicating RNA against a SARS-CoV-2 antigen. The findings by the inventors also provide the basis for a monocistronic self-replicating RNA against a SARS-CoV-2 antigen. Furthermore, the findings by the inventors provide the basis for methods of treating or preventing or delaying progression of a disease or disorder (e.g., a SARS-COV-2 infection, COVID-19 and/or acute respiratory distress syndrome (ARDS)) in a subject.

Accordingly, the present disclosure provides a self-replicating RNA comprising a nucleotide sequence encoding an antigen operably linked to a subgenomic (SG) promoter, wherein the antigen is from a SARS-CoV-2.

The present disclosure also provides a monocistronic self-replicating RNA comprising a nucleotide sequence encoding an antigen operably linked to a SG promoter, wherein the antigen is from a SARS-CoV-2.

In one example, the antigen is a spike (S) protein or a nucleocapsid (N) protein. In one example, the antigens are a SARS-CoV-2 N protein or a S protein from SARS-CoV-2 strain 2019-nCoV/USA-WA1/2020.

In one example, the antigen is a S protein. For example, the S protein is encoded by a sequence set forth in SEQ ID NO: 1.

In another example, the S protein is a mutant S protein.

In one example, a mutant S protein comprises a mutation in the receptor binding domain. For example, the mutation is selected from the group consisting of S438F, N439K, N440K, L441I, K444R, V445A, V445I, G446V, G446S, N450K, L452R, L452P, L455F, K458N, N460T, D467V, I468F, I468T, 1468V, E471O, 1472V, A475V, G476S, S477G, S477I, S477N, S477R, T478I, P479L, P479L, P479S, N481D, N481H, V483F, V483A, E484D, E484K, E484K, E484O, G485S, Y489H, Y489D, Y489F, Y489C, Y489N, F490L, F490S, P491R, Q493L, S494P, Y495N, T500N, N501S and Y505H, Y508H. In one example, a mutant S protein comprises a mutation in the receptor binding domain selected from the group consisting of N439K, N439L, L452R, S477N, T478I, V483A and E484D.

In one example, a mutant S protein comprises a mutation selected from the group consisting of P337S, F338L, F338C, G339D, E340K, V341I, A344S, T345S, R346K, A348S, A348T, W353R, N354D, N354K, N354S, S359N, D364Y, V367F, S373L, V382L, P384L, P384S, T385A, T393P, V395I, F400C, R403K, R403S, D405V, R408I, Q414E, Q414K, Q414P, Q414R, T415S, K417R, K417N, I418V, Y421S, Y423C, Y423F, Y423S, D427Y, R509K, V510L, V511E, V512L, L518I, H519O, A520S, A520V, P521R, P521S, A522P, A522S and D614G.

In one example, a mutant S protein comprises a mutation selected from the group consisting of L18F, D80A, T95I, Y144S, Y145N, D215G, P337S, F338L, F338C, G339D, E340K, V341I, A344S, T345S, R346K, A348S, A348T, W353R, N354D, N354K, N354S, S359N, D364Y, V367F, S373L, V382L, P384L, P384S, T385A, T393P, V395I, F400C, R403K, R403S, D405V, R408I, Q414E, Q414K, Q414P, Q414R, T415S, K417N, K417T, K417R, I418V, Y421S, Y423C, Y423F, Y423S, D427Y, S438F, N439K, N440K, L441I, K444R, V445A, V445I, G446V, G446S, N450K, L452R, L452P, L455F, K458N, N460T, D467V, I468F, I468T, 1468V, E471O, 1472V, A475V, G476S, S477G, S477I, S477N, S477R, T478I, T478K, P479L, P479S, N481D, N481H, V483F, V483A, E484D, E484K, E484K, E484O, G485S, Y489H, Y489D, Y489F, Y489C, Y489N, F490L, F490S, P491R, Q493L, S494P, Y495N, T500N, N501S, N501Y, Y505H, Y508H, R509K, V510L, V511E, V512L, L518I, H519O, A520S, A520V, P521R, P521S, A522P, A522S, A570D, D614G, P680H, P681H, A701V, T716I and D950N.

In one example, the mutant S protein: (i) lacks a furin cleavage site at the S1/S2 boundary and comprises RRAR to QQAA mutations at residues corresponding to nucleotides 682-685 of SEQ ID NO: 18; and/or (ii) lacks a furin cleavage site at the S2' site; and/or (iii) comprises D to G mutation at residue corresponding to nucleotide 614 of SEQ ID NO: 18; and/or (iv) comprises insertion of two proline residues between residues corresponding to nucleotides 986 and 987 of SEQ ID NO: 18.

In one example, the S protein lacks a furin cleavage site at the S1/S2 boundary and comprises RRAR to QQAA mutations at residues corresponding to nucleotides 682-685 of SEQ ID NO: 18. For example, the mutant S protein is encoded by a sequence set forth in SEQ ID NO: 2.

In one example, the S protein lacks a furin cleavage site at the S2' site.

In one example, the S protein comprises D to G mutation at residue corresponding to nucleotide 614 of SEQ ID NO: 18. For example, the mutant S protein is encoded by a sequence set forth in SEQ ID NO: 7.

In one example, the S protein comprises insertion of two proline residues between residues corresponding to nucleotides 986 and 987 of SEQ ID NO: 18.

In one example, the S protein (i) lacks a furin cleavage site at the S1/S2 boundary and comprises RRAR to QQAA mutations at residues corresponding to nucleotides 682-685 of SEQ ID NO: 18; and (ii) lacks a furin cleavage site at the S2' site. For example, the mutant S protein is encoded by a sequence set forth in SEQ ID NO: 5.

In one example, the S protein (i) lacks a furin cleavage site at the S1/S2 boundary and comprises RRAR to QQAA mutations at residues corresponding to nucleotides 682-685 of SEQ ID NO: 18; and (ii) comprises D to G mutation at residue corresponding to nucleotide 614 of SEQ ID NO: 18. For example, the mutant S protein is encoded by a sequence set forth in SEQ ID NO: 4.

In one example, the S protein (i) lacks a furin cleavage site at the S1/S2 boundary and comprises RRAR to QQAA mutations at residues corresponding to nucleotides 682-685 of SEQ ID NO: 18; and (ii) comprises insertion of two proline residues between residues corresponding to nucleotides 986 and 987 of SEQ ID NO: 18. For example, the mutant S protein is encoded by a sequence set forth in SEQ ID NO: 3.

In one example, the S protein (i) lacks a furin cleavage site at the S1/S2 boundary and comprises RRAR to QQAA mutations at residues corresponding to nucleotides 682-685 of SEQ ID NO: 18; and (ii) lacks a furin cleavage site at the S2' site; and (iii) comprises D to G mutation at residue corresponding to nucleotide 614 of SEQ ID NO: 18. For example, the mutant S protein is encoded by a sequence set forth in SEQ ID NO: 6.

In one example, the S protein (i) lacks a furin cleavage site at the S1/S2 boundary and comprises RRAR to QQAA mutations at residues corresponding to nucleotides 682-685 of SEQ ID NO: 18; and (ii) lacks a furin cleavage site at the S2' site; and (iii) comprises insertion of two proline residues between residues corresponding to nucleotides 986 and 987 of SEQ ID NO: 18.

In one example, the S protein (i) lacks a furin cleavage site at the S2' site; and (ii) comprises D to G mutation at residue corresponding to nucleotide 614 of SEQ ID NO: 18.

In one example, the S protein (i) lacks a furin cleavage site at the S2' site; and (ii) comprises insertion of two proline residues between residues corresponding to nucleotides 986 and 987 of SEQ ID NO: 18.

In one example, the S protein (i) lacks a furin cleavage site at the S2' site; and (ii) comprises D to G mutation at residue corresponding to nucleotide 614 of SEQ ID NO: 18; and (iii) comprises insertion of two proline residues between residues corresponding to nucleotides 986 and 987 of SEQ ID NO: 18.

In one example, the S protein (i) comprises D to G mutation at residue corresponding to nucleotide 614 of SEQ ID NO: 18; and (ii) comprises insertion of two proline residues between residues corresponding to nucleotides 986 and 987 of SEQ ID NO: 18.

In one example, the S protein (i) lacks a furin cleavage site at the S1/S2 boundary and comprises RRAR to QQAA mutations at residues corresponding to nucleotides 682-685 of SEQ ID NO: 18; and (ii) lacks a furin cleavage site at the S2' site; and (iii) comprises D to G mutation at residue corresponding to nucleotide 614 of SEQ ID NO: 18; and (iv) comprises insertion of two proline residues between residues corresponding to nucleotides 986 and 987 of SEQ ID NO: 18.

In one example, the S protein comprises a N to Y mutation at a residue corresponding to nucleotide 501 of SEQ ID NO: 18.

In one example, the S protein comprises deletion of two residues corresponding to nucleotides 69 and 70 of SEQ ID NO: 18.

In one example, the S protein comprises deletion of one residue corresponding to nucleotide 144 of SEQ ID NO: 18.

In one example, the S protein (i) comprises RRAR to QQAA mutations at residues corresponding to nucleotides 682-685 of SEQ ID NO: 18; and (ii) comprises deletion of two residues corresponding to nucleotides 69 and 70 of SEQ ID NO: 18; and (iii) comprises deletion of one residue corresponding to nucleotide 144 of SEQ ID NO: 18; and (iv) comprises a N to Y mutation at a residue corresponding to nucleotide 501 of SEQ ID NO: 18; and (v) comprises D to G mutation at residue corresponding to nucleotide 614 of SEQ ID NO: 18. For example, the mutant S protein is encoded by a sequence set forth in SEQ ID NO: 19.

In one example, the S protein comprises deletion of three residues corresponding to nucleotides 242 to 244 of SEQ ID NO: 18.

In one example, the S protein comprises a K to N mutation at a residue corresponding to nucleotide 417 of SEQ ID NO: 18.

In one example, the S protein comprises a E to K mutation at a residue corresponding to nucleotide 484 of SEQ ID NO: 18.

In one example, the S protein (i) comprises RRAR to QQAA mutations at residues corresponding to nucleotides 682-685 of SEQ ID NO: 18; and (ii) comprises deletion of three residues corresponding to nucleotides 242 to 244 of SEQ ID NO: 18; and (iii) comprises a K to N mutation at a residue corresponding to nucleotide 417 of SEQ ID NO: 18; and (iv) comprises a E to K mutation at a residue corresponding to nucleotide 484 of SEQ ID NO: 18; and (v) comprises a N to Y mutation at a residue corresponding to nucleotide 501 of SEQ ID NO: 18; and (vi) comprises D to G mutation at residue corresponding to nucleotide 614 of SEQ ID NO: 18. For example, the mutant S protein is encoded by a sequence set forth in SEQ ID NO: 20.

In one example, the S protein (i) comprises RRAR to QQAA mutations at residues corresponding to nucleotides 682-685 of SEQ ID NO: 18; and (ii) comprises deletion of two residues corresponding to nucleotides 69 and 70 of SEQ ID NO: 18; and (iii) comprises deletion of three residues corresponding to nucleotides 242 to 244 of SEQ ID NO: 18; and (iv) comprises a K to N mutation at a residue corresponding to nucleotide 417 of SEQ ID NO: 18; and (v) comprises a E to K mutation at a residue corresponding to nucleotide 484 of SEQ ID NO: 18; and (vi) comprises a N to Y mutation at a residue corresponding to nucleotide 501 of SEQ ID NO: 18; and (vii) comprises D to G mutation at residue corresponding to nucleotide 614 of SEQ ID NO: 18. For example, the mutant S protein is encoded by a sequence set forth in SEQ ID NO: 21.

In one example, the S protein comprises an A to D mutation at a residue corresponding to nucleotide 570 of SEQ ID NO: 18.

In one example, the S protein comprises a P to H mutation at a residue corresponding to nucleotide 680 of SEQ ID NO: 18.

In one example, the S protein comprises a T to I mutation at a residue corresponding to nucleotide 716 of SEQ ID NO: 18.

In one example, the S protein (i) comprises RRAR to QQAA mutations at residues corresponding to nucleotides 682-685 of SEQ ID NO: 18; and (ii) comprises deletion of two residues corresponding to nucleotides 69 and 70 of SEQ ID NO: 18; and (iii) comprises deletion of one residue corresponding to nucleotide 144 of SEQ ID NO: 18; and (iv) comprises a N to Y mutation at a residue corresponding to nucleotide 501 of SEQ ID NO: 18; and (v) comprises an A to D mutation at a residue corresponding to nucleotide 570 of SEQ ID NO: 18; and (vi) comprises D to G mutation at residue corresponding to nucleotide 614 of SEQ ID NO: 18; and (vii) comprises a P to H mutation at a residue corresponding to nucleotide 680 of SEQ ID NO: 18; and (viii) comprises a T to I mutation at a residue corresponding to nucleotide 716 of SEQ ID NO: 18. For example, the mutant S protein is encoded by a sequence set forth in SEQ ID NO: 22.

In one example, the S protein comprises a L to F mutation at a residue corresponding to nucleotide 18 of SEQ ID NO: 18.

In one example, the S protein comprises a D to A mutation at a residue corresponding to nucleotide 80 of SEQ ID NO: 18.

In one example, the S protein comprises a D to G mutation at a residue corresponding to nucleotide 215 of SEQ ID NO: 18.

In one example, the S protein comprises an A to V mutation at a residue corresponding to nucleotide 701 of SEQ ID NO: 18.

In one example, the S protein (i) comprises RRAR to QQAA mutations at residues corresponding to nucleotides 682-685 of SEQ ID NO: 18; and (ii) comprises a L to F mutation at a residue corresponding to nucleotide 18 of SEQ ID NO: 18; and (iii) comprises a D to A mutation at a residue corresponding to nucleotide 80 of SEQ ID NO: 18; and (iv) comprises a D to G mutation at a residue corresponding to nucleotide 215 of SEQ ID NO: 18; and (v) comprises deletion of three residues corresponding to nucleotides 242 to 244 of SEQ ID NO: 18; and (vi) comprises a K to N mutation at a residue corresponding to nucleotide 417 of SEQ ID NO: 18; and (vii) comprises a E to K mutation at a residue corresponding to nucleotide 484 of SEQ ID NO: 18; and (viii) comprises a N to Y mutation at a residue corresponding to nucleotide 501 of SEQ ID NO: 18; and (ix) comprises D to G mutation at residue corresponding to nucleotide 614 of SEQ ID NO: 18; and (x) comprises an A to V mutation at a residue corresponding to nucleotide 701 of SEQ ID NO: 18. For example, the mutant S protein is encoded by a sequence set forth in SEQ ID NO: 23.

In one example, the mutant S protein: (i) lacks a furin cleavage site at the S1/S2 boundary and comprises RRAR to QQAA mutations at residues corresponding to nucleotides 682-685 of SEQ ID NO: 18; and/or (ii) lacks a furin cleavage site at the S2' site; and/or (iii) comprises D to G mutation at residue corresponding to nucleotide 614 of SEQ ID NO: 18; and/or (iv) comprises insertion of two proline residues between residues corresponding to nucleotides 986 and 987 of SEQ ID NO: 18; and/or (v) comprises a N to Y mutation at a residue corresponding to nucleotide 501 of SEQ ID NO: 18; and/or (vi) comprises deletion of two residues corresponding to nucleotides 69 and 70 of SEQ ID NO: 18; and/or (vii) comprises deletion of one residue corresponding to nucleotide 144 of SEQ ID NO: 18; and/or (viii) comprises deletion of three residues corresponding to nucleotides 242 to 244 of SEQ ID NO: 18; and/or (ix) comprises a K to N mutation at a residue corresponding to nucleotide 417 of SEQ ID NO: 18; and/or (x) comprises a E to K mutation at a residue corresponding to nucleotide 484 of SEQ ID NO: 18; and/or (xi) comprises an A to D mutation at a residue corresponding to nucleotide 570 of SEQ ID NO: 18; and/or (xii) comprises a P to H mutation at a residue corresponding to nucleotide 680 of SEQ ID NO: 18; and/or (xiii) comprises a T to I mutation at a residue corresponding to nucleotide 716 of SEQ ID NO: 18; and/or (xix) comprises a L to F mutation at a residue corresponding to nucleotide 18 of SEQ ID NO: 18; and/or (xx); and/or comprises a D to A mutation at a residue corresponding to nucleotide 80 of SEQ ID NO: 18; and/or (xxi) comprises a D to G mutation at a residue corresponding to nucleotide 215 of SEQ ID NO: 18; and/or (xxii) comprises an A to V mutation at a residue corresponding to nucleotide 701 of SEQ ID NO: 18.

In one example, the mutant S protein is encoded by a sequence set forth in any one of SEQ ID NOs: 2 to 7.

In one example, the mutant S protein is encoded by a sequence set forth in SEQ ID NO: 2.

In one example, the mutant S protein is encoded by a sequence set forth in SEQ ID NO: 3.

In one example, the mutant S protein is encoded by a sequence set forth in SEQ ID NO: 4.

In one example, the mutant S protein is encoded by a sequence set forth in SEQ ID NO: 5.

In one example, the mutant S protein is encoded by a sequence set forth in SEQ ID NO: 6.

In one example, the mutant S protein is encoded by a sequence set forth in SEQ ID NO: 7.

In one example, the mutant S protein is encoded by a sequence set forth in any one of SEQ ID NOs: 2 to 7 and/or 19-23.

In one example, the mutant S protein is encoded by a sequence set forth in any one of SEQ ID NOs: 19 to 23.

In one example, the mutant S protein is encoded by a sequence set forth in SEQ ID NO: 19.

In one example, the mutant S protein is encoded by a sequence set forth in SEQ ID NO: 20.

In one example, the mutant S protein is encoded by a sequence set forth in SEQ ID NO: 21.

In one example, the mutant S protein is encoded by a sequence set forth in SEQ ID NO: 22.

In one example, the mutant S protein is encoded by a sequence set forth in SEQ ID NO: 23.

In one example, the antigen is a N protein. For example, the N protein is encoded by sequence set forth in SEQ ID NO: 8.

In one example, the SG promoter is a native SG promoter. For example, a native SG promoter is a promoter that is native to the RNA virus from which it is derived and/or based on (e.g., an alphavirus). In one example, the native SG promoter is a native alphavirus SG promoter.

In one example, the native SG promoter is a minimal SG promoter. For example, the minimal SG promoter is the minimal sequence required for initiation of transcription. In one example, the minimal native SG promoter is 49 nucleotides in length. In one example, the minimal native SG promoter is encoded by a sequence comprising or consisting of a sequence set forth in SEQ ID NO: 9.

In one example, the self-replicating RNA is from an alphavirus. For example, the alphavirus is selected from the group consisting of Semliki Forest virus (SFV), Sindbis virus (SIN), and Venezuelan equine encephalitis virus (VEE) and combinations thereof.

In one example, the self-replicating RNA is from a Semliki Forest virus (SFV).

In one example, the self-replicating RNA is from a Sindbis virus (SIN).

In one example, the self-replicating RNA is from a Venezuelan equine encephalitis virus (VEE).

In one example, the present disclosure provides a self-replicating RNA encoded by a sequence set forth in any one of SEQ ID NO: 10 to 17.

In one example, the present disclosure provides a self-replicating RNA encoded by a sequence set forth in SEQ ID NO: 10 (Co5).

In one example, the present disclosure provides a self-replicating RNA encoded by a sequence set forth in SEQ ID NO: 11 (Co6).

In one example, the present disclosure provides a self-replicating RNA encoded by a sequence set forth in SEQ ID NO: 12 (Co16).

In one example, the present disclosure provides a self-replicating RNA encoded by a sequence set forth in SEQ ID NO: 13 (Co17).

In one example, the present disclosure provides a self-replicating RNA encoded by a sequence set forth in SEQ ID NO: 14 (Co48).

In one example, the present disclosure provides a self-replicating RNA encoded by a sequence set forth in SEQ ID NO: 15 (Co49).

In one example, the present disclosure provides a self-replicating RNA encoded by a sequence set forth in SEQ ID NO: 16 (Co58).

In one example, the present disclosure provides a self-replicating RNA encoded by a sequence set forth in SEQ ID NO: 17 (Co59).

In one example, the present disclosure provides a self-replicating RNA encoded by a sequence set forth in SEQ ID NO: 24 (Co77).

In one example, the present disclosure provides a self-replicating RNA encoded by a sequence set forth in SEQ ID NO: 25 (Co78).

In one example, the present disclosure provides a self-replicating RNA encoded by a sequence set forth in SEQ ID NO: 26 (Co79).

In one example, the present disclosure provides a self-replicating RNA encoded by a sequence set forth in SEQ ID NO: 27 (Co80).

In one example, the present disclosure provides a self-replicating RNA encoded by a sequence set forth in SEQ ID NO: 28 (Co81).

The present disclosure also provides an immunogenic composition comprising the self-replicating RNA of the present disclosure. For example, the composition of the present disclosure, when administered, is capable of inducing an immune response in the subject. For example, administration of the composition induces a humoral and/or a cell-mediated immune response. In one example, the composition induces a humoral immune response in the subject. For example, the humoral immune response is an antibody-mediated immune response. In another example, the composition induces a cell-mediated immune response. For example, the cell-mediated immune response includes activation of antigen-specific cytotoxic T cells.

In one example, the immunogenic composition comprises a plurality of self-replicating RNAs, wherein each self-replicating RNA encodes a different polypeptide antigen sequence. For example, the different polypeptide antigen sequences are from the same strain of the virus (e.g., encode antigens from the same SARS-CoV-2 strain). In one example, the different polypeptide antigen sequences are from different strains of the same virus (e.g., encode an antigen from different strains of SARS-CoV-2). In one example, the different polypeptide antigen sequences are from different viruses (e.g., encode an antigen from SARS-CoV-2 and an antigen from an unrelated virus, e.g., influenza).

The present disclosure also provides a pharmaceutical composition comprising an immunogenic composition of the present disclosure and a pharmaceutically acceptable carrier. Pharmaceutically acceptable carriers suitable for use in the present disclosure will be apparent to the skilled person and/or are described herein.

In one example, the pharmaceutical composition further comprises a lipid nanoparticle (LNP), a polymeric microparticle, and an oil-in-water emulsion. For example, the self-replicating RNA is encapsulated in, bound to or adsorbed on a LNP, a polymeric microparticle, and an oil-in-water emulsion.

In one example, the pharmaceutical composition further comprises a LNP. For example, the self-replicating RNA is encapsulated in a LNP. In another example, the self-replicating RNA is bound to a LNP. In a further example, the self-replicating RNA is adsorbed on to a LNP.

In one example, the pharmaceutical composition further comprises a polymeric microparticle. For example, the self-replicating RNA is encapsulated in a polymeric microparticle. In another example, the self-replicating RNA is bound to a polymeric microparticle. In a further example, the self-replicating RNA is adsorbed on to a polymeric microparticle.

In one example, the pharmaceutical composition further comprises an oil-in-water emulsion. For example, the self-replicating RNA is encapsulated in an oil-in-water emulsion. In another example, the self-replicating RNA is bound to an oil-in-water emulsion. In a further example, the self-replicating RNA is adsorbed on to an oil-in-water emulsion. In a further example, the self-replicating RNA is resuspended in an oil-in-water emulsion.

The present disclosure also provides the immunogenic composition or the pharmaceutical composition of the disclosure for use as a vaccine.

The present disclosure further provides a polynucleotide encoding a self-replicating RNA vaccine of the disclosure. For example, the polynucleotide is DNA. In one example, the disclosure provides a DNA encoding a self-replicating RNA vaccine of the disclosure.

The present disclosure further provides the immunogenic composition or the pharmaceutical composition of the disclosure for use in the treatment or prevention or delaying progression of a disease or condition selected from the group consisting of a SARS-2-CoV-2 infection, COVID-19, ARDS and combinations thereof. For example, the disclosure provides the immunogenic composition or the pharmaceutical composition of the disclosure for use in the treatment of a SARS-2-CoV-2 infection, COVID-19, ARDS and combinations thereof. In one example, the disclosure provides the immunogenic composition or the pharmaceutical composition of the disclosure for use in the prevention of a SARS-2-CoV-2 infection, COVID-19, ARDS and combinations thereof. In another example, the disclosure provides the immunogenic composition or the pharmaceutical composition of the disclosure for use in delaying the progression of a SARS-2-CoV-2 infection, COVID-19, ARDS and combinations thereof.

In one example, the present disclosure provides the immunogenic composition or the pharmaceutical composition of the disclosure for use in the treatment or prevention or delaying progression of COVID-19. For example, the disclosure provides the immunogenic composition or the pharmaceutical composition of the disclosure for use in the treatment of COVID-19. In another example, the disclosure provides the immunogenic composition or the pharmaceutical composition of the disclosure for use in the prevention of COVID-19. In a further example, the disclosure provides the immunogenic composition or the pharmaceutical composition of the disclosure for use in delaying the progression of COVID-19.

In one example, the present disclosure provides the immunogenic composition or the pharmaceutical composition of the disclosure for use in treatment or prevention or delaying progression of a SARS-CoV-2 infection. For example, the disclosure provides the immunogenic composition or the pharmaceutical composition of the disclosure for use in the treatment of a SARS-CoV-2 infection. In another example, the disclosure provides the immunogenic composition or the pharmaceutical composition of the disclosure for use in the prevention of a SARS-CoV-2 infection. In a further example, the disclosure provides the immunogenic composition or the pharmaceutical composition of the disclosure for use in delaying the progression of a SARS-CoV-2 infection.

In one example, the present disclosure provides the immunogenic composition or the pharmaceutical composition of the disclosure for use in the treatment or prevention or delaying progression of ARDS. For example, the disclosure provides the immunogenic composition or the pharmaceutical composition of the disclosure for use in the treatment of ARDS. In another example, the disclosure provides the immunogenic composition or the pharmaceutical composition of the disclosure for use in the prevention of ARDS. In a further example, the disclosure provides the immunogenic composition or the pharmaceutical composition of the disclosure for use in delaying progression of ARDS.

The present disclosure provides a method of treating or preventing or delaying progression of a disease or condition in a subject, the method comprising administering the immunogenic composition or the pharmaceutical composition of the present disclosure to a subject in need thereof. In one example, the disclosure provides a method of treating a disease or condition in a subject, the method comprising administering the immunogenic composition or the pharmaceutical composition of the present disclosure to a subject in need thereof. In another example, the disclosure provides a method of preventing a disease or condition in a subject, the method comprising administering the immunogenic composition or the pharmaceutical composition of the present disclosure to a subject in need thereof. In a further example, the disclosure provides a method of delaying progression of a disease or condition in a subject, the method comprising administering the immunogenic composition or the pharmaceutical composition of the present disclosure to a subject in need thereof.

In one example, the present disclosure provides use of a self-replicating RNA of the disclosure in the manufacture of a medicament for treating or preventing or delaying progression of a disease or condition in a subject in need thereof. For example, the disclosure provides use of a self-replicating RNA of the disclosure in the manufacture of a medicament for treating a disease or condition in a subject in need thereof. In another example, the disclosure provides use of a self-replicating RNA of the disclosure in the manufacture of a medicament for preventing a disease or condition in a subject in need thereof. In a further example, the disclosure provides use of a self-replicating RNA of the disclosure in the manufacture of a medicament for delaying progression of a disease or condition in a subject in need thereof.

In one example, the subject suffers from a disease or condition. In one example, the subject has been diagnosed as suffering from a disease or condition. In one example, the subject is receiving treatment for a disease or condition.

In one example, the disease or condition is selected from the group consisting of a SARS-CoV-2 infection, COVID-19, ARDS and combinations thereof. In one example, the disease or condition is a SARS-CoV-2 infection. In another example, the disease or condition is COVID-19. In a further example, the disease or condition is ARDS. In one example, the ARDS is associated with a SARS-CoV-2 infection and/or COVID-19. For example, the disease or condition is ARDS. In one example, the ARDS is associated with a SARS-CoV-2 infection. In another example, the disease or condition is ARDS. In one example, the ARDS is associated with COVID-19.

In one example of any method described herein, the self-replicating RNA of the present disclosure is administered before or after the development of a SARS-CoV-2 infection, COVID-19 and/or ARDS in a subject. In one example of any method described herein, the self-replicating RNA of the present disclosure is administered before the development of a SARS-CoV-2 infection, COVID-19 and/or ARDS in a subject. In one example of any method described herein, the self-replicating RNA of the present disclosure is administered after the development of a SARS-CoV-2 infection, COVID-19 and/or ARDS in a subject.

In one example of any method described herein, the self-replicating RNA of the present disclosure is administered after the detection of a SARS-CoV-2 infection, COVID-19 and/or ARDS in a subject. In one example of any method described herein, the self-replicating RNA of the present disclosure is administered after the detection of a SARS-CoV-2 infection. In another example, the self-replicating RNA of the present disclosure is administered after the detection of a SARS-CoV-2 infection but prior to the development of COVID-19. In a further example of any method described herein, the self-replicating RNA of the present disclosure is administered after the detection of COVID-19. In one example of any method described herein, the self-replicating RNA of the present disclosure is administered after the detection of COVID-19 but prior to the development of ARDS. In another example of any method described herein, the self-replicating RNA of the present disclosure is administered after the detection of ARDS.

In one example, the subject is at risk of developing COVID-19 or ARDS. For example, the subject is at risk of developing COVID-19. In a further example, the subject is at risk of developing ARDS.

In one example, the composition of the present disclosure is administered in an amount sufficient to reduce the severity of or prevent onset of one or more symptoms of a SARS-CoV-2 infection, COVID-19 and/or ARDS. Symptoms of a SARS-CoV-2 infection, COVID-19 and/or ARDS will be apparent to the skilled person and/or are described herein.

The present disclosure provides a method of inducing an immune response in a subject, comprising administering the self-replicating RNA, the immunogenic composition or the pharmaceutical composition of the present disclosure to a subject in need thereof.

The present disclosure also provides use of the self-replicating RNA, the immunogenic composition or the pharmaceutical composition of the present disclosure in the manufacture of a medicament for inducing an immune response in a subject in need thereof.

In one example, the self-replicating RNA, the immunogenic composition or the pharmaceutical composition of the present disclosure induces a humoral and/or a cell-mediated immune response. In one example, the composition induces a humoral immune response in the subject. For example, the humoral immune response is an antibody-mediated immune response. For example, production of neutralizing antibodies. In another example, the composition induces a cell-mediated immune response. For example, the cell-mediated immune response includes activation of antigen-specific cytotoxic T cells. For example, the T cells are CD4 T cells and/or CD8 T cells. In one example, the T cells are CD4 T cells. In another example the T cells are CD8 T cells. In a further example, the T cells are CD4 and CD8 T cells.

In one example, administration of the self-replicating RNA, the immunogenic composition or the pharmaceutical composition of the present disclosure induces a CD4 T cell mediated immune response.

In one example, administration of the self-replicating RNA, the immunogenic composition or the pharmaceutical composition of the present disclosure induces a CD8 T cell mediated immune response.

In one example, administration of the self-replicating RNA, the immunogenic composition or the pharmaceutical composition of the present disclosure induces a CD4 and a CD8 T cell mediated immune response.

In one example, the CD4 T cell mediated immune response is a Th0, a Th1 and/or a Th2 response. For example, the CD4 T cell mediated immune response is a Th0 response. In another example, the CD4 T cell mediated immune response is a Th1 response. In a further example, the CD4 T cell mediated immune response is a Th2 response. In one example, the CD4 T cell mediated immune response is a Th0 and Th1 response. In another example, the CD4 T cell mediated immune response is a Th0 and Th2 response. In a further example, the CD4 T cell mediated immune response is a Th1 and Th2 response. In another example, the CD4 T cell mediated immune response is a Th0, Th1 and Th2 response.

In one example, the Th0 response cytokines express interleukin 2 (IL2+) and/or tumor necrosis factor alpha (TNFa+); and/or are negative for interferon gamma (IFNg-), IL5- and/or IL13-. For example, the cytokine is IL2+. In another example, the cytokine is TNFa+. In one example, the cytokine is IFNg-. In another example, the cytokine is IL5-. In a further example, the cytokine is IL13-.

In one example, the Th1 response cytokines express interferon gamma (IFNg+); and/or are negative for IL5- and/or IL13-. For example, the cytokine is IFNg+. In another example, the cytokine is IL5-. In a further example, the cytokine is IL13-.

In one example, the Th2 response cytokines express IL5+ and/or IL13+; and/or are negative for IFNg. For example, the cytokine is IL5+. In a further example, the cytokine is IL13+. For example, the cytokine is IFNg-.

The present disclosure also provides a polynucleotide that encodes the self-replicating RNA of the present disclosure. For example, the polynucleotide is a recombinant DNA. In one example, the recombinant DNA is a plasmid. In one example, the plasmid comprises a sequence set forth in any one of SEQ ID NO: 10 to 17.

The present disclosure also provides a kit comprising at least one self-replicating RNA of the disclosure, optionally in a delivery system and/or a pharmaceutically acceptable carrier or diluent, packaged with instructions for use in treating or preventing or delaying progression of a disease or disorder (e.g., a SARS-CoV-2 infection, COVID-19 and/or ARDS) in a subject.

The present disclosure also provides a kit comprising at least one self-replicating RNA of the disclosure, optionally in a delivery system and/or a pharmaceutically acceptable carrier or diluent, packaged with instructions to administer the RNA to a subject who is suffering from or at risk of suffering from a disease or disorder (e.g., a SARS-CoV-2 infection, COVID-19 and/or ARDS).

In one example, the self-replicating RNA, the immunogenic composition or the pharmaceutical composition of the disclosure is supplied in a vial. In another example, the self-replicating RNA, the immunogenic composition or the pharmaceutical composition of the disclosure is supplied in a syringe.

### BRIEF DESCRIPTION OF THE DRAWINGS

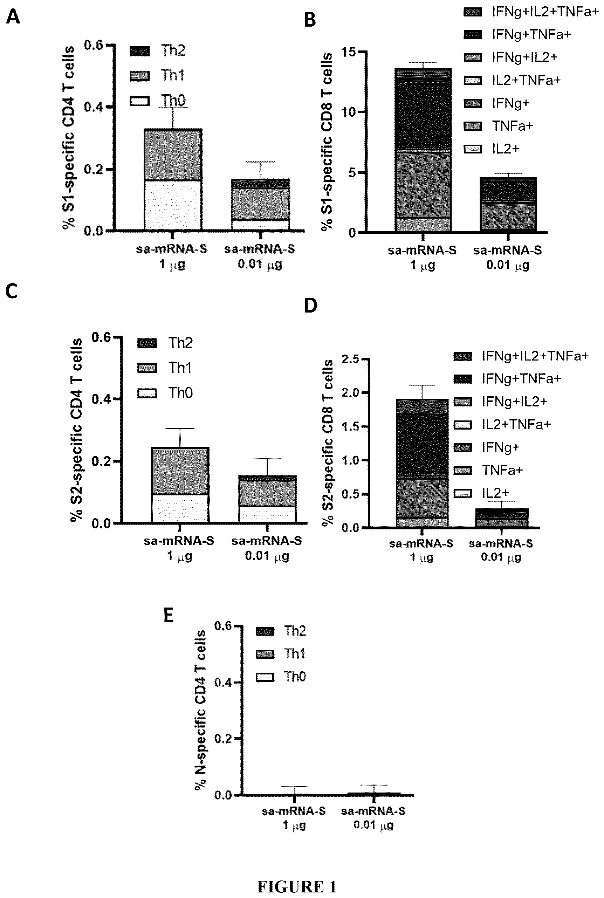

**Figure 1** is a series of graphical representations showing antigen-specific T cells induced by Co16. The net (antigen-specific) % cytokine-producing CD4 and CD8 T cells induced are shown for **(A)** S1-specific CD4 T cells, **(B)** S1-specific CD8 T cells **(C)** S2-specific CD4 T cells, **(D)** S2-specific CD8 T cells, and **(E)** N-specific CD4 T cells.

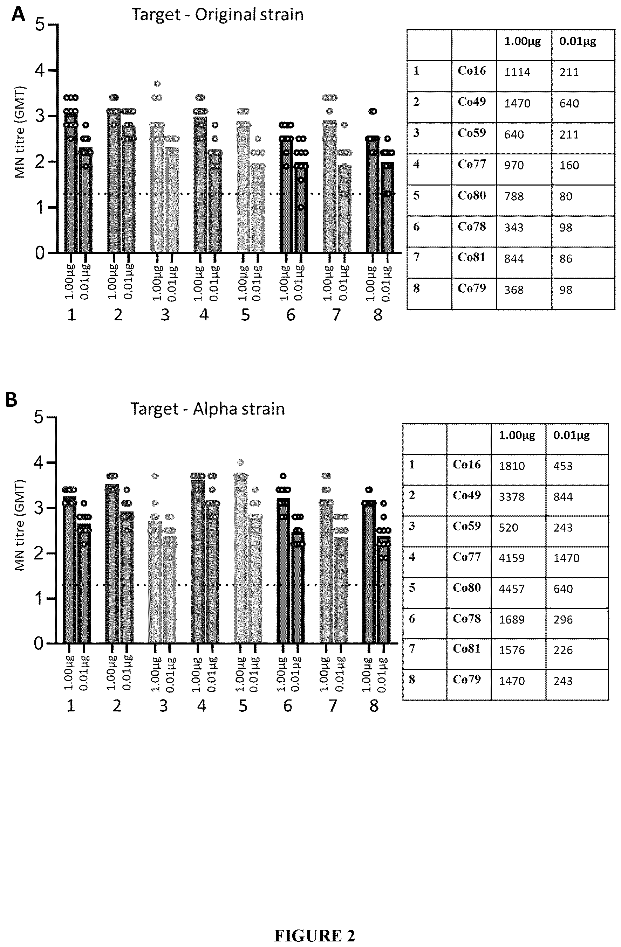

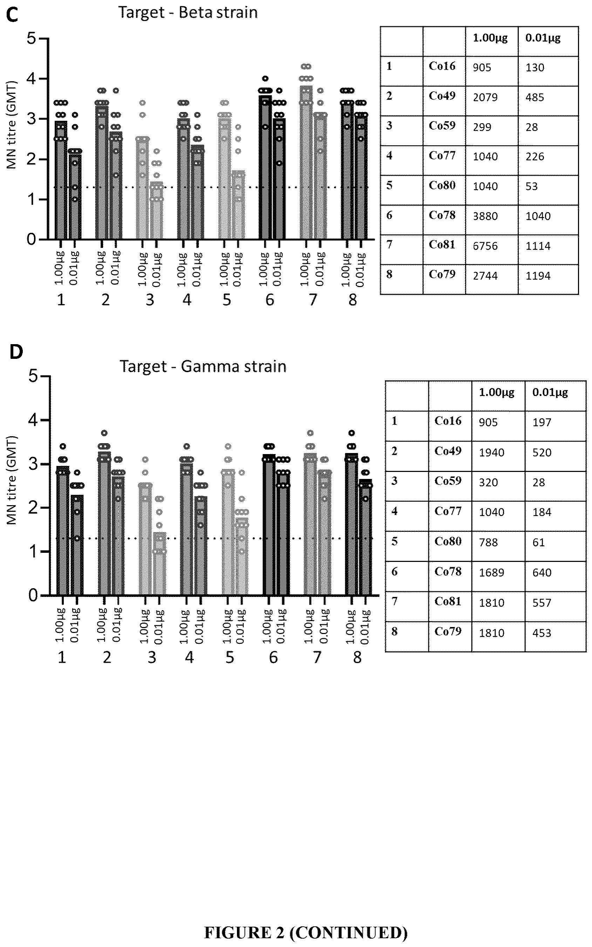

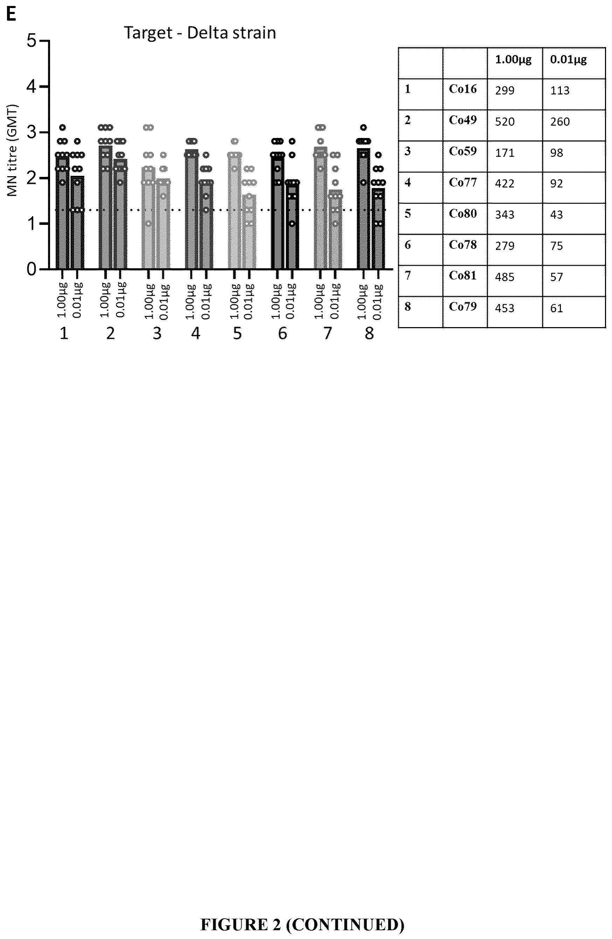

**Figure 2** is a series of graphical representations showing neutralisation capabilities of the constructs against **(A)** the reference Whuan sequence; **(B)** the alpha variant (B.1.1.7; UK strain); **(C)** beta variant (B.1.351; South Aftrican strain); **(D)** gamma variant (P.1; Brazillian strain); and **(E)** delta variant (B.1.617.2; Indian strain).

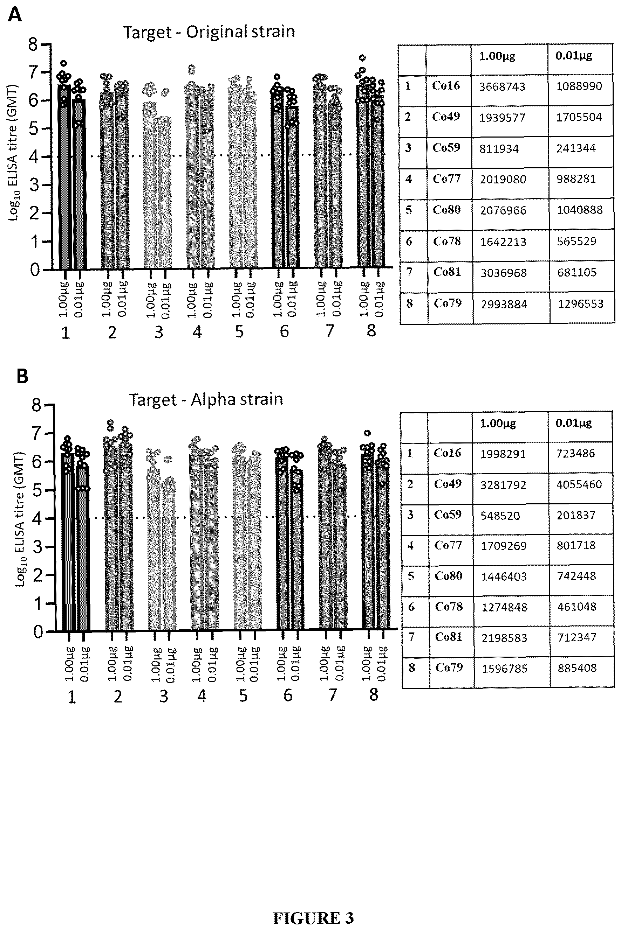

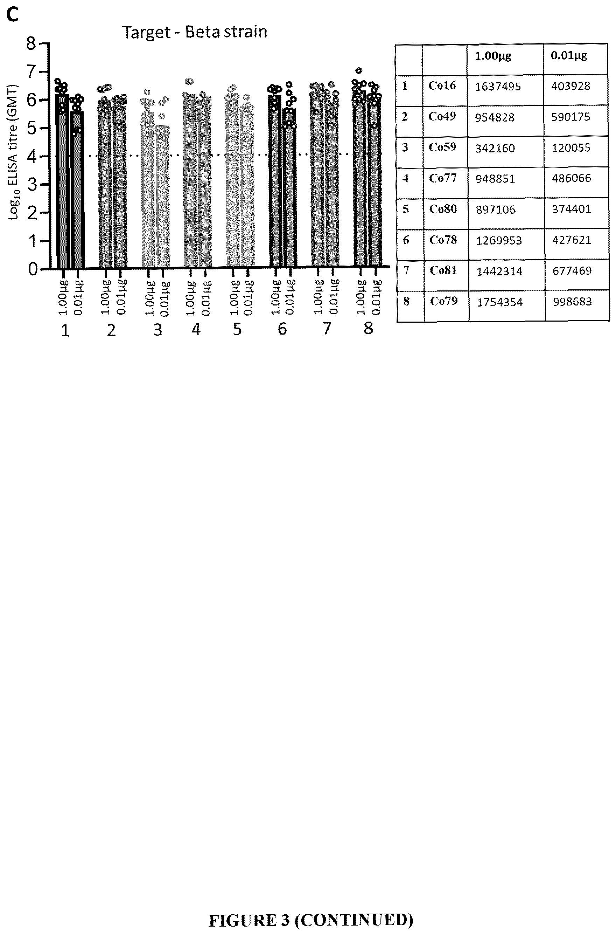

**Figure 3** is a series of graphical representations showing all constructs generated total Ig responses at high and low doses against **(A)** the reference Whuan sequence; **(B)** the alpha variant (B.1.1.7; UK strain); (C) beta variant (B.1.351; South Aftrican strain); **(D)** gamma variant (P.1; Brazillian strain); and **(E)** delta variant (B.1.617.2; Indian strain).

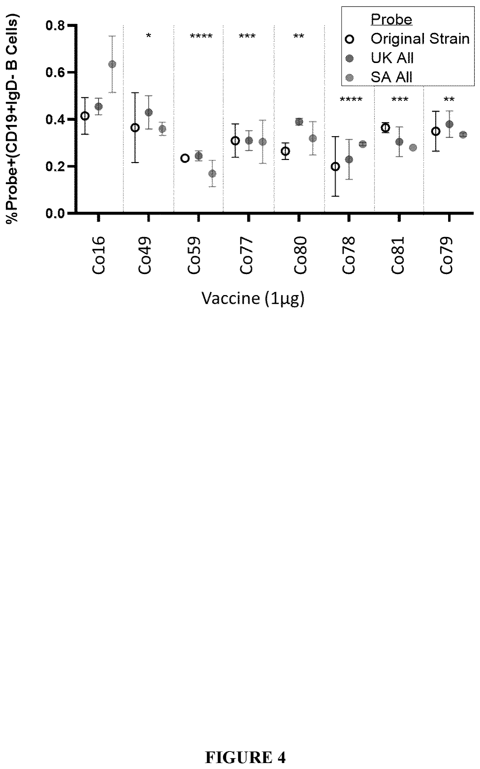

**Figure 4** is a graphical representation showing all constructs generated S-specific B cells that reacted with all variant B-cell receptor specific probes. Non-specific controls (i.e., no bait and negative control HA H1) showed low levels of background binding.

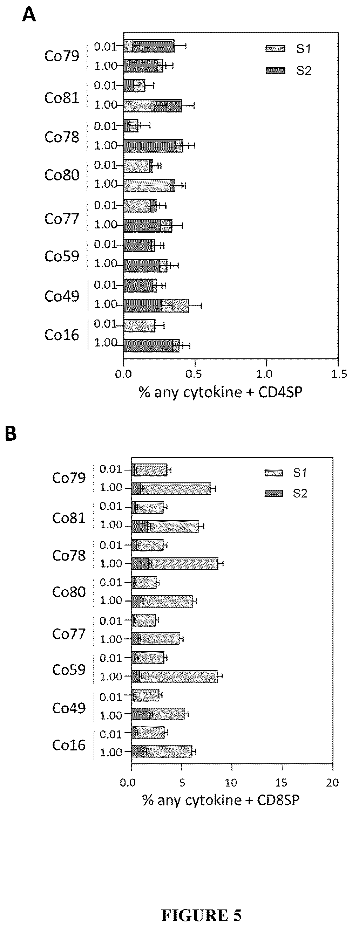

**Figure 5** is a series of graphical representations showing all constructs induced antigen-specific **(A)** CD4 and **(B)** CD8 T cells reactive with S1 and S2 epitopes.

### KEY TO SEQUENCE LISTING

| | |
|---|---|
| **SEQ ID NO: 1** | Nucleotide sequence of SARS-CoV-2 spike (S) protein full length wt (cleavable) |
| **SEQ ID NO: 2** | Nucleotide sequence of SARS-CoV-2 mutated spike (S) protein uncleavable (S1/S2 RRAR to QQAA mutation) |
| **SEQ ID NO: 3** | Nucleotide sequence of SARS-CoV-2 spike (S) protein uncleavable (S1/S2 RRAR to QQAA mutation and 986P/987P mutation) |
| **SEQ ID NO: 4** | Nucleotide sequence of SARS-CoV-2 spike (S) protein uncleavable (S1/S2 RRAR to QQAA mutation and D614G mutation) |
| **SEQ ID NO: 5** | Nucleotide sequence of SARS-CoV-2 spike (S) protein uncleavable (S1/S2 RRAR to QQAA mutation and S2' mutation) |
| **SEQ ID NO: 6** | Nucleotide sequence of SARS-CoV-2 spike (S) protein uncleavable (S1/S2 RRAR to QQAA mutation and D614G mutation and S2' mutation) |
| **SEQ ID NO: 7** | Nucleotide sequence of SARS-CoV-2 spike (S) protein cleavable (D614G mutation) |
| **SEQ ID NO: 8** | Nucleotide sequence of SARS-CoV-2 nucleocapsid (N) protein full length wt |
| **SEQ ID NO: 9** | Nucleotide sequence of alphavirus native subgenomic promoter |
| **SEQ ID NO: 10** | Nucleotide sequence of construct Co5 |
| **SEQ ID NO: 11** | Nucleotide sequence of construct Co6 |
| **SEQ ID NO: 12** | Nucleotide sequence of construct Co16 |
| **SEQ ID NO: 13** | Nucleotide sequence of construct Co17 |
| **SEQ ID NO: 14** | Nucleotide sequence of construct Co48 |
| **SEQ ID NO: 15** | Nucleotide sequence of construct Co49 |
| **SEQ ID NO: 16** | Nucleotide sequence of construct Co58 |
| **SEQ ID NO: 17** | Nucleotide sequence of construct Co59 |
| **SEQ ID NO: 18** | Amino acid sequence of SARS-CoV-2 S protein full length wt |
| **SEQ ID NO: 19** | Nucleotide sequence of SARS-CoV-2 spike (S) protein uncleavable (RRAR→QQAA; Δ69-70; ΔY144; N501Y; D614G) |
| **SEQ ID NO: 20** | Nucleotide sequence of SARS-CoV-2 spike (S) protein uncleavable (RRAR→QQAA; Δ242-244; K417N; E484K; N501Y; D614G) |
| **SEQ ID NO: 21** | Nucleotide sequence of SARS-CoV-2 spike (S) protein uncleavable (RRAR→QQAA; Δ69-70; Δ242-244; K417N; E484K; N501Y; D614G) |
| **SEQ ID NO: 22** | Nucleotide sequence of SARS-CoV-2 spike (S) protein uncleavable (RRAR→QQAA; Δ69-70; ΔY144; N501Y; A570D; D614G; P680H; T716I) |
| **SEQ ID NO: 23** | Nucleotide sequence of SARS-CoV-2 spike (S) protein uncleavable (RRAR→QQAA; L18F; D80A; D215G; Δ242-244; K417N; E484K; N501Y; D614G; A701V) |
| **SEQ ID NO: 24** | Nucleotide sequence of construct Co77 |
| **SEQ ID NO: 25** | Nucleotide sequence of construct Co78 |
| **SEQ ID NO: 26** | Nucleotide sequence of construct Co79 |
| **SEQ ID NO: 27** | Nucleotide sequence of construct Co80 |
| **SEQ ID NO: 28** | Nucleotide sequence of construct Co81 |
| **SEQ ID NO: 29** | Nucleotide sequence of 5'UTR of VEEV |
| **SEQ ID NO: 30** | Nucleotide sequence of 3'UTR of SINV |
| **SEQ ID NO: 31** | GC-rich element |
| **SEQ ID NO: 32** | GC-rich element |
| **SEQ ID NO: 33** | GC-rich element |
| **SEQ ID NO: 34** | Histone stem loop sequence |
| **SEQ ID NO: 35** | Kozak consensus sequence |
| **SEQ ID NO: 36** | Kozak consensus sequence |
| **SEQ ID NO: 37** | Poly-A sequence |

### DETAILED DESCRIPTION

### General

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or groups of compositions of matter.

Those skilled in the art will appreciate that the present disclosure is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific examples described herein, which are intended for the purpose of exemplification only. Functionally-equivalent products, compositions and methods are clearly within the scope of the present disclosure.

Any example of the present disclosure herein shall be taken to apply *mutatis mutandis* to any other example of the disclosure unless specifically stated otherwise. Stated another way, any specific example of the present disclosure may be combined with any other specific example of the disclosure (except where mutually exclusive).

Any example of the present disclosure disclosing a specific feature or group of features or method or method steps will be taken to provide explicit support for disclaiming the specific feature or group of features or method or method steps.

Unless specifically defined otherwise, all technical and scientific terms used herein shall be taken to have the same meaning as commonly understood by one of ordinary skill in the art (for example, in cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al. Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

The term "and/or", e.g., "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As used herein the term "derived from" shall be taken to indicate that a specified integer may be obtained from a particular source albeit not necessarily directly from that source. Similarly, the term "based on" shall be taken to indicate that a specified integer may be developed or used from a particular source albeit not necessarily directly from that source.

### Selected Definitions

As used herein, the term "self-replicating RNA" refers to a construct based on an RNA virus that has been engineered to allow expression of heterologous RNA and proteins. Self-replicating RNA (e.g., in the form of naked RNA) can amplify in host cells leading to expression of the desired gene product in the host cell.

As used herein, the term "monocistronic" in reference to the self-replicating RNA, refers to a RNA that encodes one polypeptide.

The term "naked" as used herein refers to nucleic acids that are substantially free of other macromolecules, such as lipids, polymers and proteins. A "naked" nucleic acid, such as a self-replicating RNA, is not formulated with other macromolecules to improve cellular uptake. Accordingly, a naked nucleic acid is not encapsulated in, absorbed on, or bound to a LNP, a liposome, a polymeric microparticle or an oil-in-water emulsion.

As used herein, the term "nucleotide sequence" or "nucleic acid sequence" will be understood to mean a series of contiguous nucleotides (or bases) covalently linked to a phosphodiester backbone. By convention, sequences are presented from the 5' end to the 3' end, unless otherwise specified.

As used herein, the term "antigen" refers to a molecule or structure containing one or more epitopes that induce, elicit, augment or boost a cellular and/or humoral immune response. Antigens can include, for example, proteins and peptides from a pathogen such as a virus, bacteria, fungus, protozoan, plant or from a tumour.

As used herein, the term "operably linked to" means positioning a subgenomic promoter relative to a nucleic acid such that expression of the nucleic acid is controlled or regulated by the element.

As used herein, the term "subgenomic promoter" (SG; also known as 'junction region' promoter) refers to a promoter that directs the expression of a heterologous nucleotide sequence, regulating protein expression.

The term "polypeptide" or "polypeptide chain" will be understood to mean a series of contiguous amino acids linked by peptide bonds. For example, a protein shall be taken to include a single polypeptide chain i.e., a series of contiguous amino acids linked by peptide bonds or a series of polypeptide chains covalently or non-covalently linked to one another (i.e., a polypeptide complex). The series of polypeptide chains can be covalently linked using a suitable chemical or a disulfide bond. Examples of noncovalent bonds include hydrogen bonds, ionic bonds, Van der Waals forces, and hydrophobic interactions.

The term "recombinant" shall be understood to mean the product of artificial genetic recombination.

As used herein, the terms "disease", "disorder" or "condition" refers to a disruption of or interference with normal function, and is not to be limited to any specific condition, and will include diseases or disorders.

As used herein, a subject "at risk" of developing a disease or condition may or may not have detectable disease or symptoms of disease, and may or may not have displayed detectable disease or symptoms of disease prior to the treatment according to the present disclosure. "At risk" denotes that a subject has one or more risk factors, which are measurable parameters that correlate with development of the disease or condition, as known in the art and/or described herein.

As used herein, the terms "treating", "treat" or "treatment" include administering a RNA or composition described herein to thereby reduce or eliminate at least one symptom of a specified disease or condition.

As used herein, the term "preventing", "prevent" or "prevention" includes providing prophylaxis with respect to occurrence or recurrence of a specified disease or condition in an individual. An individual may be predisposed to or at risk of developing the disease but has not yet been diagnosed with the disease.

As used herein, the phrase "delaying progression of" includes reducing or slowing down the progression of the disease or condition in an individual and/or at least one symptom of a disease or condition.

An "effective amount" refers to at least an amount effective, at dosages and for periods of time necessary, to achieve the desired result. For example, the desired result may be a therapeutic or prophylactic result. An effective amount can be provided in one or more administrations. In some examples of the present disclosure, the term "effective amount" is meant an amount necessary to effect treatment of a disease or condition as hereinbefore described. In some examples of the present disclosure, the term "effective amount" is meant an amount necessary to effect a change associated with a disease or condition as hereinbefore described. The effective amount may vary according to the disease or condition to be treated or factor to be altered and also according to the weight, age, racial background, sex, health and/or physical condition and other factors relevant to the mammal being treated. Typically, the effective amount will fall within a relatively broad range (e.g. a "dosage" range) that can be determined through routine trial and experimentation by a medical practitioner. Accordingly, this term is not to be construed to limit the disclosure to a specific quantity, e.g., weight or number of RNA. The effective amount can be administered in a single dose or in a dose repeated once or several times over a treatment period.

A "therapeutically effective amount" is at least the minimum concentration required to effect a measurable improvement of a particular disease or condition. A therapeutically effective amount herein may vary according to factors such as the disease state, age, sex, and weight of the patient, and the ability of the RNA of the present disclosure to elicit a desired response in the individual. A therapeutically effective amount is also one in which any toxic or detrimental effects of the RNA are outweighed by the therapeutically beneficial effects.

As used herein, the term "prophylactically effective amount" shall be taken to mean a sufficient quantity of the RNA of the disclosure to prevent or inhibit or delay the onset of one or more detectable symptoms of a disease or disorder as described herein.

As used herein, the term "subject" shall be taken to mean any animal including humans, for example a mammal. Exemplary subjects include but are not limited to humans and non-human primates. For example, the subject is a human.

### Self-replicating RNA

The present disclosure provides a self-replicating RNA (also known as a replicon). For example, the present disclosure provides a monocistronic self-replicating RNA.

The skilled person will understand that the self-replicating RNA of the present disclosure is based on the genomic RNA of RNA viruses. The RNA should be positive (+)-stranded so that it can be directly translated after delivery to a cell without the need for intervening replication steps (e.g., reverse transcription). Translation of the RNA results in the production of non-structural proteins (NSPs) which combine to form a replicase complex (i.e., an RNA-dependent RNA polymerase). The complex then amplifies the original RNA, producing both antisense and sense transcripts, resulting in production of multiple daughter RNAs which may subsequently be translated and transcribed, enhancing overall protein expression.

In one example, the self-replicating RNA of the present disclosure comprises the non-structural proteins of the RNA virus, the 5' and 3' untranslated regions (UTRs) and the native subgenomic promoter.

In one example, the self-replicating RNA comprises one or more non-structural proteins of the RNA virus. For example, the RNA comprises at least one or more genes selected from the group consisting of a viral replicase (or viral polymerase), a viral protease, a viral helicase and other non-structural viral proteins. For example, the self-replicating RNA comprises a viral replicase (or viral polymerase).

It will be apparent to the skilled person that RNA suitable for use in the present disclosure may also include a 5' untranslated region (5'-UTR), a 3' untranslated region (3'UTR), and/or a coding or translating sequence. In addition, the RNA may comprise a 5' cap structure, a chain terminating nucleotide, a stem loop (e.g., a histone stem loop), a 3' tailing sequence (e.g., a polyadenylation signal or one or more polyA tails. In another example, the self-replicating RNA comprises a 5'- and a 3'-end UTR of the RNA virus. It will be apparent to the skilled person that the terms 5' and a 3'UTR also encompasses the terms 5' and 3' conserved sequence elements (CSE). In one example, the self-replicating RNA comprises a 5'- and a 3'-end CSE.

The self-replicating RNA of the present disclosure cannot induce production of infectious viral particles. For example, the self-replicating RNA of the present disclosure does not comprise viral genes encoding structural proteins necessary for production of viral particles.

In one example, the self-replicating RNA is derived from or based on an alphavirus. Suitable alphaviruses will be apparent to the skilled person and/or described herein.

In another example, the self-replicating RNA is derived from or based on a virus other than an alphavirus, for example, a positive-stranded RNA virus. Suitable positive-stranded RNA viruses suitable for use in the present disclosure will be apparent to the skilled person and include, for example, a picornavirus, a flavivirus, a rubivirus, a pestivirus, a hepacivirus, a calicivirus, or a coronavirus.

### Alphavirus

In one example, the self-replicating RNA of the present disclosure is derived from (or based on) an alphavirus

Alphaviruses are the sole genus in the *Togaviridae* family and are an enveloped virus with a positive-sense, single-stranded RNA genome. The skilled person will understand that the alphavirus genome comprises two open reading frames (ORFs), non-structural and structural. The first ORF encodes four non-structural proteins (NSP1, NSP2, NSP3 and NSP4) necessary for transcription and replication of viral RNA. The second encodes three structural proteins: the core nucleocapsid protein C, and the envelope proteins P62 and E1, which associate as a heterodimer. The viral membrane-anchored surface glycoproteins are responsible for receptor recognition and entry into target cells through membrane fusion.

In one example, the self-replicating RNA of the present disclosure comprises a viral replicase (or viral polymerase). For example, the viral replicase is an alphavirus replicase, such as an alphavirus protein NSP4.

In one example, the self-replicating RNA of the present disclosure does not encode one or more alphavirus structural proteins (e.g., capsid and/or envelope glycoproteins). For example, the self-replicating RNA is unable to produce RNA-containing alphavirus virions (i.e., infectious viral particles).

In one example, the self-replicating RNA comprises a native alphavirus SG promoter. For example, the native alphavirus SG promoter is a minimal SG promoter (i.e., the minimal sequence required for initiation of transcription) and comprises a sequence set forth in SEQ ID NO: 9.

The skilled person will be aware of alphaviruses suitable for use in the present disclosure. Exemplary alphaviruses include, but are not limited to, Venezuelan equine encephalitis virus (VEE; e.g., Trinidad donkey, TC83CR), Semliki Forest virus (SFV), Sindbis virus (SIN), Ross River virus, Western equine encephalitis virus, Eastern equine encephalitis virus, Chikungunya virus, S.A. AR86 virus, Everglades virus, Mucambo virus, Barmah Forest virus, Middelburg virus, Pixuna virus, O'nyong-nyong virus, Getah virus, Sagiyama virus, Bebaru virus, Mayaro virus, Una virus, Aura virus, Whataroa virus, Banbanki virus, Kyzylagach virus, Highlands J virus, Fort Morgan virus, Ndumu virus, and Buggy Creek virus. The term alphavirus may also include chimeric alphaviruses (e.g., as described by Perri et al, (2003) J. Virol. 77(19): 10394-403) that contain genome sequences from more than one alphavirus.

### Subgenomic Promoter

The present disclosure provides a self-replicating RNA comprising a nucleotide sequence encoding an antigen operably linked to a SG promoter.

SG promoters (also known as 'junction region' promoters) suitable for use in the present disclosure will be apparent to the skilled person and/or are described herein.

In one example, the SG promoter is derived from or based on an alphavirus SG promoter. For example, the SG promoter is a native alphavirus SG promoter. In one example, the native SG promoter is a minimal SG promoter. For example, the minimal SG promoter is the minimal sequence required for initiation of transcription.

### 5' untranslated region (5 'UTR)

In one example, the self-replicating RNA comprises a 5'- UTR of the RNA virus.

As used herein, the term "5'-untranslated region" or "5'-UTR" refers to a non-coding region of an mRNA located at the 5'end of the translation initiation sequence (AUG).

In one example, the 5'UTR is a 5'UTR of a Venezuelan equine encephalitis virus (VEEV) or modified forms thereof. For example, the 5'UTR comprises a sequence set forth in SEQ ID NO: 29.

In one example, the 5'UTR comprises at least one microRNA binding site, an AU rich element (ARE), a GC-rich element, a stem loop, and combinations thereof.

### microRNA binding site

As used herein, the term "microRNA binding site" refers to a sequence within a polyncleotide (e.g. within a DNA or RNA transcript) that has sufficient complementarity to all or one region of a miRNA to interact, associate or bind to the microRNA (miRNA).

As used herein, the term "microRNA" or "miRNA" refers to 19-25 nucleotide long non-coding RNAs that bind to the 5'-UTR of polynucleotides and down-regulate gene expression (e.g. by inhibiting translation). The presence of microRNA binding site(s) in the 5'UTR of the present disclosure can function to inhibit translation of the 5'-UTR.

Suitable miRNA binding sites for use in the present disclosure will be apparent to the skilled person and/or described herein.

In one example, the miRNA binding site comprises a binding site for tissue specific microRNA or those regulating biological processes. For example, miRNA of the liver (miR-122), muscle (miR-133, miR-206, miR-208), endothelial cells (miR-17-92, miR-126), myeloid cells (miR-142-3p, miR-142-5p, miR-16, miR-21, miR-223, miR-24, miR-27), adipose tissue (let-7, miR-30c), heart (miR-id, miR-149), kidney (miR-192, miR-194, miR-204), and lung epithelial cells (let-7, miR-133, miR-126). For example, microRNA that regulate biological processes such as angiogenesis (miR-132). Further exemplifying miRNA and miRNA binding sites are disclosed in US patent application US14/043,927.

### AU rich element (ARE)

As used herein, the term "AU rich element (ARE)" or "AU rich elements (AREs)" refers to a region of a nucleotide sequence comprising stretches of Adeonisine (A) and Uridine (U). Exemplary AREs include, for example, ARE from cytoplasmic myc (c-myc), myoblast determination protein 1 (myoD), c-Jun, Myogenin, granulocyte-macrophage colony-stimulating factor (GM-CSF) and tumour necrosis factor alpha (TNF-α), or a combination thereof.

In one example, the ARE comprises a human antigen R or "HuR" (also known as Elav11) specific binding site. HuR is known to bind AREs increasing the stability of the mRNA.

### GC-rich element

As used herein, the term "GC-rich element" refers to a nucleotide sequence with a high amount of Guanine (G) and/or Cytosine (C) compared to Adenine (A) and Thymine(T)/Uracil(U). The presence of GC-rich elements in a polynucleotide (e.g. mRNA) can stabilise the mRNA.

In one example, the GC-rich element comprises a sequence of 3, or 4, or 5, or 6, or 7, or 8, or 9, or 10, or 11, or 12, or 13, or 14, or 15, or 16, or 17, or 18, or 19, or 20, or 21, or 22, or 23, or 24, or 25, or 26, or 27, or 28, or 29, or 30 nuceleotides in length.

In one example, the GC-rich element comprises between 30% and 40%, or 40% and 50%, or 50% and 60%, or 60% and 70% cytosine. For example, the GC-rich element comprises between 30% and 40% cytosine. For example, the GC-rich element comprises between 40% and 50% cytosine. For example, the GC-rich element comprises between 50% and 60% cytosine. For example, the GC-rich element comprises between 60% and 70% cytosine.

In one example, the GC-rich element comprises 30%, or 40%, or 50%, or 60%, or 70% cytosine. For example, the GC-rich element comprise 30% cytosine. For example, the GC-rich element comprises 40% cytosine. For example, the GC-rich element comprises 50% cytosine. For example, the GC-rich element comprises 60% cytosine. For example, the GC-rich element comprises 60% cytosine. For example, the GC-rich element comprises 70% cytosine.

In one example, the GC-rich element is at least 50% cytosine.

In one example, the GC-rich element is at least 60% cytosine.

In one example, the GC-rich element is at least 70% cytosine.

In one example, the GC-rich element comprises a nucleotide sequence CCCCGGCGCC. In another example, the GC-rich element comprises a nucleotide sequence CCCCGGC. In a further example, the GC-rich element comprises a nucleotide sequence GCGCCCCGCGGCGCCCCGCG.

In one example, the GC-rich element comprises a nucleotide sequence set forth in SEQ ID NO: 31 to 33. In one example, the GC-rich element comprises a nucleotide sequence set forth in SEQ ID NO: 31. In another example, the GC-rich element comprises a nucleotide sequence set forth in SEQ ID NO: 32. In a further example, the GC-rich element comprises a nucleotide sequence set forth in SEQ ID NO: 33.

### Stem loop

As used herein, the term "stem loop" refers to a nucleotide sequence comprising an intramolecular base pairing of two neighboured entirely or partially reverse complementary sequences to form a stem-loop. A stem-loop can occur in single-stranded DNA or, more commonly, in RNA. The stem loop can also be referred to as a hairpin or hairpin loop which usually consists of a stem and a terminal loop within a consecutive sequence, wherein the stem is formed by two neighboured entirely or partially reverse complementary sequences separated by a short sequence which builds the loop into a stem-loop structure.

The stability of the paired stem loop is determined by the length, the number of mismatched or bulges it contains, and the nucleotide composition of the paired region.

In one example, a loop of the stem loop is between 3 and 10 nucleotides in length. For example, the loop of the stem loop is between 3 and 8, or 3 and 7, or 3 and 6, or 4 and 5 nucleotides in length.

In one example, the loop of the stem loop is 4 nucleotides in length.

In one example, the stem loop is a histone stem loop. For example, the histone stem loop comprises or consist of a nucleotide sequence set for in SEQ ID NO: 34.

### Kozak consensus sequence

As used herein, the term "Kozak consensus sequence" refers to a nucleotide sequence identified in eukaryotic genes that facilitates the translation of the gene by containing a start codon (also referred to as a translation initiation codon) which is recognised by a ribosome.

Exemplary Kozak consensus sequence are known in the art and/or described herein. In one example, the Kozak consensus sequence is set forth in SEQ ID NO: 35. In another example, the Kozak consensus sequence is set forth in SEQ ID NO: 36. In one example, the Kozak consensus sequence is ACCATGG. In another example, the Kozak consensus sequence is ACCATG.

### 3 'untranslated region (3'-UTR)

In one example, the self-replicating RNA comprises a 3'- UTR of the RNA virus.

As used herein, the term "3'-UTR" refers to a region of an mRNA located at the 3'end of the the translation termination codon (i.e. stop codon).

In one example, the 3'UTR is a 3'UTR of a Sindbis virus (SINV) or modified forms thereof. For example, the 3'UTR comprises a sequence set forth in SEQ ID NO: 30.

In one example, the 3'-UTR of the present disclosure further comprises at least one microRNA binding site, an AU rich element (ARE), a GC-rich element, a triple helix, a stem loop, one or more stop codons or a combination thereof.

### Stop codon

As used herein, the term "stop codon" refers to a trinucleotide sequence within a mRNA that signals the stop of protein synthesis by a ribosome.

In one example, the polynucleotide of the present disclosure comprises at least one stop codon at the 5'end of a 3'-UTR. For example, the stop codon is selected from UAG, UAA, and UGA.

In one example, the polynucleotide comprises two consecutive stop codons comprising a sequence UGAUGA.

In one example, the polynucleotide comprises two consecutive stop codons comprising a sequence UAAUAG.

### 3 ' tailing sequence

The RNA of the present disclosure comprises one or more 3' tailing sequences located at the 3'end of the 3'UTR.

As described herein, the term "3' tailing sequence" or "3' tailing sequences" refers to a nucleotide sequence (e.g. polyadenylation signal) which induces the addition of non-encoded nucleotides to the 3'end of a mRNA or a nucleotide sequence (e.g. poly-A sequence) located at the 3' end of a mRNA. A skilled person will appreciate that the 3'tailing sequence and/or products of the 3'tailing sequence in a mRNA functions to stabilise the mRNA and/or prevent the mRNA from degradation.

As used herein, the term "interrupting linker" in reference to a poly-A or poly-C sequence of the present disclosure refers to a single nucleotide or nucleotide sequence which are linked to, and interrupt, a stretch of consecutive adenosine or cytosine nucleotides in the poly-A or poly-C sequence. For example, the interrupting linker in a poly-A sequence is a single nucleotide or a nucleotide sequence consisting or comprising a nucleotide other than an adenosine nucleotide. For example, the interrupting linker in a poly-C sequence is a single nucleotide or a nucleotide sequence consisting or comprising a nucleotide other than a cytosine nucleotide.

In one example, the one or more 3' tailing sequences are selected from the group consisting of a poly-A sequence, polyadenylation signal, a G-quadruplex, a poly-C sequence, a stem loop and combinations thereof.

### Poly-A sequence

As used herein, the term "polyA sequence" refers to a nucleotide sequence of Adenine (A) located at the 3'end of a mRNA. In the context of the present disclosure, the polyA sequence may be located within the mRNA or DNA (e.g. a DNA plasmid serving as a template for generating the mRNA by transcription of the vector).

Suitable poly-A sequence for use in the present disclosure will be apparent to the skilled person and/or are described herein. In one example, the poly-A sequence comprises consecutive (i.e. one after the other) adenosine nucleotides of any length (e.g. to 10 to 300). For example, the poly-A sequence comprises 36 consecutive adenosine nucleotides. In one example, the poly-A sequence comprises a sequence set forth in SEQ ID NO: 37.

In one example, the poly-A sequence comprises consecutive adenosine nucleotides separated by one or more interrupting linkers. In one example, the poly-A sequence comprises consecutive adenosine nucleotides without an interrupting linker.

### Polyadenylation signal

As used herein, the term "polyadenylation signal" refers to a nucleotide sequence which induces polyadenylation. Polyadenylation is typically understood to be the addition of a polyA sequence to a RNA (e.g. to a premature mRNA to generate a mature mRNA). The polyadenylation signal may be located within a nucleotide sequence at the 3'-end of the polynucleotide (e.g. mRNA) to be polyadenylated.

Suitable polyadenylation signal for use in the present disclosure will be apparent to the skilled person and/or described herein.

In one example, the polyadenylation signal comprises a hexamer consisting of Adenine and Uracil/Thymidine nucleotides. In one example, the hexamer sequence comprises or consists of AAUAAA.

In one example, the 3'tailing sequence comprises a polyadenylation signal but does not comprise a polyA sequence.

### G-quadruplex

As used herein, the term "G-quadruplex" or "G4" refers to a nucleotide sequence rich in guanine residues which forms a four stranded secondary structure. For example, the G-quadruplex is a cyclic hydrogen bonded array of four guanine nucleotides formed by G-rich sequences in both DNA and RNA.

In one example, the 3' tailing sequence comprises a polyA sequence and a G-quadruplex. For example, the 3' tailing sequence comprises a polyA sequence linked to a G-quadruplex to produce a polyA-G quartet.

### Poly-C sequence

As used herein, the term "poly-C sequence" refers to a nucleotide sequence of Cytosine (C) located at the 3'end of a mRNA. In the context of the present disclosure, the polyC sequence may be located within the mRNA or DNA (e.g. a DNA plasmid serving as a template for generating the mRNA by transcription of the vector).

Suitable poly-C sequence for use in the present disclosure will be apparent to the skilled person and/or are described herein.

In one example, the one or more 3' tailing sequences comprises one or more poly-C sequences each comprising between 10 and 300 consecutive cytosine nucleotides. For example, the one or more poly-C sequences each comprises between 10 and 20, or 20 and 30, or 30 and 40, or 40 and 50, or 50 and 60, or 60 and 70, or 70 and 80, or 80 and 90, or 90 and 100, or 100 and 125, or 125 and 150, or 150 and 175, or 175 and 200, or 200 and 225, or 225 and 250, or 250 and 275, or 275 and 300 consecutive cytosine nucleotides. For example, the one or more poly-C sequence each comprises 10, or 20, or 30, or 40, or 50, or 60, or 70, or 80, or 90, or 100, or 125, or 150, or 175, or 200, or 225, or 250, or 275, or 300 consecutive cytosine nucleotides.

In one example, the one or more poly-C sequences is separated by an interrupting linker. For example, the fourth nucleotide sequence comprising the one or more 3'tailing sequences comprises, in order of 5' to 3': consecutive cytosine nucleotides, an interrupting linker, and further consecutive cytosine nucleotides.

In one example, the interrupting linker is from 10 to 50, or 50 to 100, or 100 to 150 nucleotides in length. For example, the interrupting linker is 1, or 2, or 3, or 4, or 5, or 6, or 7, or 8, or 9, or 10, or 11, or 12, or 13, or 14, or 15, or 16, or 17, or 18, or 19, or 20, or 25, or 30, or 35, or 40, or 45, or 50, or 55, or 60, or 65, or 70, or 75, or 80, or 85, or 90, or 95, or 100, or 110, or 120, or 130, or 140, or 150 nucleotides in length.

### 5'Cap

In one example, the self-replicating RNA comprises a 5'terminal cap structure.

As used herein, the term "5'cap structure" refers to a structure at the 5' terminal end of a mRNA involved in nuclear export and binds a mRNA Cap Binding Protein (CBP). The 5'cap structure is known to stabilise mRNA through association of CBP with poly(A) binding protein to form a mature mRNA. Accordingly, the presence of a 5'cap structure in the mRNA of the present disclosure can further increase the stability of the mRNA compared to a mRNA without the 5'cap.

Exemplary 5'cap structure includes, for example, anti-reverse cap analogue (ARCA), N7,2'-0-dimethyl-guanosine (mCAP), inosine, N1-methyl-guanosine, 2'fluoro-guanosine, 7-deaza-guanosine, 8-oxo-guanosine, 2-amino-guanosine, LNA-guanosine, 2-azido-guanosine, N6,2'-O-dimethyladenosine, 7-methylguanosine (m7G), Cap1, and Cap2.

Typically, an endogenous mRNA is 5'capped with a guanosine through a (5)'-ppp-(5)'-triphosphate linkage attached to the 5'terminal nucleotide of the mRNA. The guanosine cap can then be methylated to a 7-methylguanosine (m7G) generating a 7mG(5')ppp(5')N,pN2p (Cap0 structure), where N represents the first and second 5'terminal nucleotide of the mRNA. The cap0 structure can be further 2'-O-methylated to produce 7mG(5')ppp(5')NlmpNp (Cap1), and/or 7mG(5')-ppp(5')NlmpN2mp (Cap2).

In one example, the polynucleotide of the present disclosure comprises an endogenous cap.

As used herein, the term "endogenous cap" refers to a 5'cap synthesised in a cell. For example, endogenous cap is a natural 5'cap or a wild-type 5'cap. For example, the endogenous cap is a CapO, Cap1, or Cap2 structure.

In one example, the polynucleotide of the present disclosure comprises an analog of an endogenous cap (also referred to as cap analog).

As used herein, the term "analogue thereof" in the context of an endogenous cap or "cap analog" refers to a synthetic 5'cap. The cap analog can be used to produce 5'capped mRNA in *in* vitro transcription reactions. Cap analogs may be chemically (i.e. non-ezymatically) or enzymatically synthesized and/or linked to a nucleotide (e.g. 5'terminal nucleotide of an mRNA). Exemplary cap analogs are commercially available and include, for example, 3"-O-Me-m7G(5')ppp(5')G, G(5')ppp(5')A, G(5')ppp(5')G, m7G(5')ppp(5')A, m7G(5')ppp(5')G (New England BioLabs). In one example, the cap analog is N7,3'-O-dimethyl-guanosine-5'-triphosphate-5'-guanosine (i.e. anti-reverse cap analogue (ARCA)).

In one example, the 5'cap structure is a non-hydrolyzable cap structure. The non-hydrolyzable cap structure can prevent decapping of the mRNA and increase the half-life of the mRNA.

In one example, the non-hydrolyzable cap structure comprises a modified nucleotide selected from a group consisting or a α-thio-guanosine nucleotide, α-methylphosphonate, seleno-phosphate, and a combination thereof. In one example, the modified nucleotide is linked to the 5'end of the mRNA through an α-phosphorothiate linkage. Methods of linking the modified nucleotide to the 5'end of the mRNA will be apparent to the skilled person. For example, using a Vaccinia Capping Enzyme (New England Biolabs).

### Antigens

The self-replicating RNA of the present disclosure comprises a nucleotide sequence that encodes an antigen (e.g., a pathogenic antigen). For example, the antigen can induce an immune response in the subject.

In one example, the self-replicating RNA of the present disclosure comprises a nucleotide sequence that encodes an antigen from SARS-CoV-2.

### Methods of Production

Suitable methods for the production of a self-replicating RNA of the present disclosure will be apparent to the skilled person and/or described herein.

In one example, the self-replicating RNA is produced using a plasmid DNA. The skilled person will understand that plasmid DNA is relatively stable. Briefly, competent bacterial cells (e.g., *Escherichia coli)* cells are transformed with a DNA plasmid encoding a self-replicating RNA of the present disclosure. Individual bacterial colonies are isolated and the resultant plasmid DNA amplified in *E. coli* cultures.

In one example, the plasmid DNA is isolated following fermentation. For example, the plasmid DNA is isolated using a commercially available kit (e.g., Maxiprep DNA kit), or other routine methods known to the skilled person. Following isolation, plasmid DNA is linearized by restriction digest (i.e., using a restricting enzyme). Restriction enzymes are removed using methods known in the art, including for example phenol/chloroform extraction and ethanol precipitation.

In one example, mRNA is made by *in vitro* transcription from a linearized DNA template using an RNA polymerase (e.g., T7 RNA polymerase). Following *in vitro* transcription, the DNA template is removed by DNase digestion. The skilled person will understand that synthetic mRNA capping is performed to correct mRNA processing and contribute to stabilization of the mRNA. In one example, the mRNA is enzymatically 5'-capped. For example, the 5' cap is a cap0 structure or a cap1 structure. In one example, the 5' cap is a cap0 structure, for example, the 5'-cap (i.e., cap0) consists of an inverted 7-methylguanosine connected to the rest of the mRNA via a 5'-5' triphosphate bridge. In one example, the 5' cap is a cap1 structure, for example, the 5'-cap (i.e., cap1) consists of the cap0 with an additional methylation of the 2'O position of the initiating nucleotide.

In one example, the mRNA is purified. Various methods for purifying mRNA will be apparent to the skilled person. For example, the mRNA is purified using lithium chloride (LiCl) precipitation. In another example, the mRNA is purified using tangential flow filtration (TFF). Following purification, the mRNA is resuspended in e.g., nuclease-free water.

### Compositions

The present disclosure provides an immunogenic composition comprising a self-replicating RNA of the present disclosure.

The present disclosure also provides a pharmaceutical composition comprising an immunogenic composition of the present disclosure and a pharmaceutically acceptable carrier.

It will be apparent to the skilled person and/or described herein, that the self-replicating RNA of the present disclosure may be present as naked RNA or in combination with lipids, polymers or other delivery system that facilitates entry into the cells.

### Delivery systems

In one example, the pharmaceutical composition of the present disclosure further comprises a LNP, a polymeric microparticle and an oil-in-water emulsion. For example, the self-replicating RNA is encapsulated in, bound to or adsorbed on a LNP, a polymeric microparticle, or an oil-in-water emulsion.

### Lipid Nanoparticles

In one example, the pharmaceutical composition of the present disclosure further comprises a LNP.

It will be apparent that the term "lipid nanoparticle" refers to any lipid composition, including, but not limited to, liposomes or vesicles, where an aqueous volume is encapsulated by amphipathic lipid bilayers (e.g., single; unilamellar or multiple; multilamellar) micelle-like lipid nanoparticles having a non-aqueous core and solid lipid nanoparticles, wherein solid lipid nanoparticles lack lipid bilayers.

Lipid nanoparticles suitable for use in the present disclosure will be apparent to the skilled person and/or are described herein. The lipids can have an anionic, cationic or zwitterionic hydrophilic head group.

In one example, the lipid nanoparticle comprises a PEG-lipid, a sterol structural lipid and/or a neutral lipid. In one example, the lipid nanoparticle further comprises a cationic lipid. In one example, the lipid nanoparticle does not comprise a cationic lipid.

In one example, the LNP comprises a PEG-lipid. For example, the PEG-lipid is selected from the group consisting of PEG-c-DMG, PEG-DMG, PEG-DLPE, PEG-DMPE, PEG-DPPC, a PEG-DSPE lipid and combinations thereof.

In one example, the LNP comprises a structural lipid. For example, the structural lipid is selected from the group consisting of cholesterol fecosterol, sitosterol, campesterol, stigmasterol, brassicasterol, ergosterol, tomatidine, tomatine, ursolic acid and alpha-tocopherol and combinations thereof.

In one example, the LNP comprises a neutral lipid. Exemplary phospholipids (anionic or zwitterionic) for use in the present disclosure include, for example, phosphatidylethanolamines, phosphatidylcholines, phosphatidylserines, and phosphatidylglycerols. For example, the neutral lipid is selected from the group consisting of 1,2-distearoyl-sn-glycero-3-phosphocholine (DSPC), 1,2-dioleoyl-sn-glycero-3-phosphoethanolamine (DOPE), 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine (DSPE), 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), and sphingomyelin and combinations thereof.

In one example, the LNP comprises a cationic lipid. Exemplary cationic lipids include, but are not limited to, dioleoyl trimethylammonium propane (DOTAP), 1,2-distearyloxy-N,N-dimethyl-3-aminopropane (DSDMA), 1 ,2-dioleyloxy- N,Ndimethyl-3-aminopropane (DODMA), 1 ,2-dilinoleyloxy-N,N-dimethyl-3- aminopropane (DLinDMA), 1 ,2-dilinolenyloxy-N,N-dimethyl-3-aminopropane (DLenDMA), 2,5-bis((9z,12z)-octadeca-9,12,dien-l-yloxyl)benzyl-4-(dimethylamino)butnoate (LKY750). In one example, the phospholipid is 2,5-bis((9z,12z)-octadeca-9,12,dien-1-yloxyl)benzyl-4-(dimethylamino)butnoate (LKY750). Exemplary zwitterionic lipids include, but are not limited to, acyl zwitterionic lipids and ether zwitterionic lipids, such as dipalmitoylphosphatidylcholine (DPPC), dioleoylphosphatidylcholine (DOPC) and dodecylphosphocholine. The lipids can be saturated or unsaturated.

### Polymeric microparticles

In one example, the pharmaceutical composition of the present disclosure further comprises a polymeric microparticle.

The skilled person will be aware that various polymers can form microparticles to encapsulate or adsorb the self-replicating RNA of the present disclosure. It will be apparent that use of a substantially non-toxic polymer means that particles are safe, and the use of a biodegradable polymer means that the particles can be metabolised after delivery to avoid long-term persistence. Useful polymers are also sterilisable, to assist in the preparation of pharmaceutical grade formulations.

Exemplary non-toxic and biodegradable polymers include, but are not limited to, poly(α- hydroxy acids), polyhydroxy butyric acids, polylactones (including polycaprolactones), polydioxanones, polyvalerolactone, polyorthoesters, polyanhydrides, polycyanoacrylates, tyrosine-derived polycarbonates, polyvinyl-pyrrolidinones or polyester-amides, and combinations thereof.

### Oil-in-water cationic emulsions

In one example, the pharmaceutical composition of the present disclosure further comprises an oil-in-water cationic emulsion.

Suitable oils for use in an oil-in-water emulsion will be apparent to the skilled person and/or are described herein. For example, the emulsion comprises one or more oils derived, for example, from an animal (e.g., fish) or a vegetable source (e.g., nuts, seeds, grains). The skilled person will recognise that biocompatible and biodegradable oils are preferentially used. Exemplary animal oils (i.e., fish oils) include cod liver oil, shark liver oils, and whale oil. Exemplary vegetable oils include peanut oil, coconut oil, olive oil, soybean oil, jojoba oil, safflower oil, cottonseed oil, sunflower seed oil, sesame seed oil, corn oil.

In addition to the oil, the oil-in-water emulsion also comprises a cationic lipid to facilitate formation and stabilisation of the emulsion. Suitable cationic lipids will be apparent to the skilled person and/or are described herein. Exemplary cationic lipids include, but are not limited to, limited to: l,2-dioleoyloxy-3-(trimethylammonio)propane (DOTAP), 3'-[N-(N',N'-Dimethylaminoethane)-carbamoyl] Cholesterol (DC Cholesterol), dimethyldioctadecyl-ammonium (DDA), 1,2-Dimyristoyl-3-Trimethyl-AmmoniumPropane (DMTAP), dipalmitoyl[C16:0]trimethyl ammonium propane (DPTAP) and distearoyltrimethylammonium propane (DSTAP).

In some examples, the oil-in-water emulsion also comprises a non-ionic surfactant and/or a zwitterionic surfactant. The skilled person will be aware of surfactants suitable for use in the present disclosure. Exemplary surfactants include, but are not limited to: the polyoxyethylene sorbitan esters surfactants (e.g., polysorbate 20 and polysorbate 80) and copolymers of ethylene oxide (EO), propylene oxide (PO), and/or butylene oxide (BO).

### Pharmaceutically acceptable carrier

Suitably, in compositions or methods for administration of the self-replicating RNA of the disclosure to a subject, the self-replicating RNA is combined with a pharmaceutically acceptable carrier as is understood in the art. Accordingly, one example of the present disclosure provides a composition (e.g., a pharmaceutical composition) comprising the self-replicating RNA of the disclosure (and any delivery system) combined with a pharmaceutically acceptable carrier.

In general terms, by "carrier" is meant a solid or liquid filler, binder, diluent, encapsulating substance, emulsifier, wetting agent, solvent, suspending agent, coating or lubricant that may be safely administered to any subject, e.g., a human. Depending upon the particular route of administration, a variety of acceptable carriers, known in the art may be used, as for example described in Remington's Pharmaceutical Sciences (Mack Publishing Co. N.J. USA, 1991).

A self-replicating RNA of the present disclosure is useful for parenteral, topical, oral, or local administration, intramuscular administration, aerosol administration, or transdermal administration, for prophylactic or for therapeutic treatment. In one example, the self-replicating RNA is administered parenterally, such as intramuscularly, subcutaneously or intravenously. For example, the self-replicating RNA is administered intramuscularly.

Formulation of a self-replicating RNA to be administered will vary according to the route of administration and formulation (e.g., solution, emulsion, capsule) selected. An appropriate pharmaceutical composition comprising a self-replicating RNA to be administered can be prepared in a physiologically acceptable carrier. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. A variety of appropriate aqueous carriers are known to the skilled artisan, including water, buffered water, buffered saline, polyols (e.g., glycerol, propylene glycol, liquid polyethylene glycol), dextrose solution and glycine. Intravenous vehicles can include various additives, preservatives, or fluid, nutrient or electrolyte replenishers (See, generally, Remington's Pharmaceutical Science, 16th Edition, Mack, Ed. 1980). The compositions can optionally contain pharmaceutically acceptable auxiliary substances as required to approximate physiological conditions such as pH adjusting and buffering agents and toxicity adjusting agents, for example, sodium acetate, sodium chloride, potassium chloride, calcium chloride and sodium lactate. The self-replicating RNA can be stored in the liquid stage or can be lyophilized for storage and reconstituted in a suitable carrier prior to use according to art-known lyophilization and reconstitution techniques.

The optimum concentration of the active ingredient(s) in the chosen medium can be determined empirically, according to procedures known to the skilled artisan, and will depend on the ultimate pharmaceutical formulation desired.

Upon formulation, compositions of the present disclosure will be administered in a manner compatible with the dosage formulation and in such amount as is therapeutically/prophylactically effective. The dosage ranges for the administration of the molecule of the disclosure are those large enough to produce the desired effect. For example, the composition comprises an effective amount of the self-replicating RNA. In one example, the composition comprises a therapeutically effective amount of the self-replicating RNA. In another example, the composition comprises a prophylactically effective amount of the self-replicating RNA.

The dosage should not be so large as to cause adverse side effects. Generally, the dosage will vary with the age, condition, sex and extent of the disease in the patient and can be determined by one of skill in the art. The dosage can be adjusted by the individual physician in the event of any complication.

Dosage can vary from about 0.1 mg/kg to about 300 mg/kg, e.g., from about 0.2 mg/kg to about 200 mg/kg, such as, from about 0.5 mg/kg to about 20 mg/kg, in one or more dose administrations daily, for one or several days.

In some examples, the self-replicating RNA is administered at an initial (or loading) dose which is higher than subsequent (maintenance doses). For example, the self-replicating RNA is administered at an initial dose of between about 10mg/kg to about 30mg/kg. The self-replicating RNA is then administered at a maintenance dose of between about 0.0001mg/kg to about 10mg/kg. The maintenance doses may be administered every 7-35 days, such as, every 7 or 14 or 28 days.

In some examples, a dose escalation regime is used, in which the self-replicating RNA is initially administered at a lower dose than used in subsequent doses. This dosage regime is useful in the case of subject's initially suffering adverse events

In the case of a subject that is not adequately responding to treatment, multiple doses in a week may be administered. Alternatively, or in addition, increasing doses may be administered.

A subject may be retreated with the self-replicating RNA, by being given more than one exposure or set of doses, such as at least about two exposures of the binding protein, for example, from about 2 to 60 exposures, and more particularly about 2 to 40 exposures, most particularly, about 2 to 20 exposures.

In one example, the subject is treated with a first dose of the self-replicating RNA on day 0 and is subsequently treated with a second dose of the self-replicating RNA on day 21. For example, the first and second doses are administered 21 days (or 3 weeks) apart.

In another example, the subject is treated with a first dose of the self-replicating RNA on day 0 and is subsequently treated with a second dose of the self-replicating RNA on day 28. For example, the first and second doses are administered 28 days (or 4 weeks) apart.

In one example, any retreatment may be given when signs or symptoms of disease return.

In another example, any retreatment may be given at defined intervals. For example, subsequent exposures may be administered at various intervals, such as, for example, about 24-28 weeks or 48-56 weeks or longer. For example, such exposures are administered at intervals each of about 24-26 weeks or about 38-42 weeks, or about 50-54 weeks.

In the case of a subject that is not adequately responding to treatment, multiple doses in a week may be administered. Alternatively, or in addition, increasing doses may be administered.

In another example, for subjects experiencing an adverse reaction, the initial (or loading) dose may be split over numerous days in one week or over numerous consecutive days.

Administration of the self-replicating RNA according to the methods of the present disclosure can be continuous or intermittent, depending, for example, on the recipient's physiological condition, whether the purpose of the administration is therapeutic or prophylactic, and other factors known to skilled practitioners. The administration of the self-replicating RNA may be essentially continuous over a preselected period of time or may be in a series of spaced doses, e.g., either during or after development of a condition.

### Screening Assays

Suitable methods for selecting a self-replicating RNA of the present disclosure are available to those skilled in the art. Assays may be conducted to assess the efficiency and efficacy of the RNA including, for example, serology and immune responses.

### Antigen expression

In one example, the self-replicating RNA is assessed for expression of the gene of interest.

For example, antigen expression is detected using antibodies against the gene of interest. In one example, the number of cells positive for antigen expression is measured by e.g., fluorescence-activated cell sorting (FACS). In another example the mean fluorescence intensity (MFI) is determined using e.g., FACS. In a further example, the specific potency value or the probability of successful transfection per unit mass of RNA is calculated.

### Microneutralization Assay

In one example, the self-replicating RNA (naked and/or formulated) is assessed for antibody responses. For example, the self-replicating RNA is assessed using a microneutralisation assay. Methods of performing a microneutralization assay will be apparent to the skilled person. In one example, the microneutralization assay is a short form assay. For one example, a virus fluorescent focus-based microneutralization assay is performed. In another example, the microneutralization assay is a long form assay.

### Antigen Specific T cell Responses

In one example, the self-replicating RNA is assessed for its ability to induce antigen specific T cell responses. Methods of assessing induction of antigen specific T cell responses will be apparent to the skilled person and/or are described herein.

For example, antigen-specific T cell detection is performed on splenic cultures. Briefly, splenocyte cultures are established in T cell medium and cell cultures are either stimulated with antigenic peptides or unstimulated. In one example, antigen-specific T cell responses are determined using flow cytometry.

### Neutralising assays

The self-replicating RNA of the disclosure may be screened *in vitro* for their ability to bind to a SARS-CoV-2 S protein RBD and neutralises binding of the S protein RBD to ACE2. Suitable assays will be apparent to the skilled person and include, for example, a Vero microneutralisation assay, a sVNT assay, or a psuedovirus neutralisation assay (using e.g., HEK-293T cells or HeLa-ACE2 cells).

In one example, the neutralization assay is a Vero microneutralization assay. Briefly, SARS-Cov-2 wild-type virus is passaged in Vero cells (i.e., the Vero lineage isolated from kidney epithelial cells extracted from an African green monkey). Serial two-fold dilutions of a test protein are incubated with 100 TCID₅₀ (i.e., median tissue culture infectious dose) of SARS-CoV-2 for 1 hour and residual virus infectivity is assessed in Vero cells; viral cytopathic effect is read, for example, on day 5. The neutralising antibody titre is calculated using the Reed/Muench method as previously described (Houser et al., 2016; Subbarao et al 2004).

In one example, the neutralization assay is a surrogate neutralization test (sVNT). Briefly, the wells of a plate are coated with hACE2 protein in carbonate-bicarbonate coating buffer (e.g., pH 9.6). HRP-conjugated SARS-CoV-2 and HRP-conjugated SARS-CoV-RBD pre-incubated with test proteins is added to the hACE2 at different concentrations and incubated, for example, for 1h at room temperature. Unbound HRP conjugated antigens are removed by washing. Colorimetric signal is developed on the enzymatic reaction of HRP with chromogenic substrate, e.g., 3,3',5,5'-tetramethylbenzidine (TMB). In one example, the absorbance reading at 450 nm and 570 nm is acquired.

In one example, the neutralisation is a psuedovirus neutralisation assay. Briefly, HIV reporter virus pseudotyped with SARS-2-Spike protein is produced by co-transfection of SARS-2-COV-2 spike plasmids together with a viral backbone plasmid (e.g., pDR-NL Δenv FLUC) into e.g., HEK-293T cells. Pseudovirus is harvested post transfection and clarified by filtration. Virus stock titres, reported as Relative Luciferase Units infectious dose (RLU), are calculated by limiting dilution infections in Hela-hACE2 cells measuring luciferase activity as a read-out for viral infection.

### Methods of Treatment or Prevention

The present disclosure provides methods of using the immunogenic composition or the pharmaceutical composition of the present disclosure as a vaccine.

The present disclosure also provides methods of treating or preventing or delaying progression of a disease or condition in a subject comprising administering the immunogenic composition or the pharmaceutical composition of the present disclosure. For example, the disease or condition is selected from the group consisting of SARS-CoV-2 infection, COVID-19, ARDS and combinations thereof.

### Coronavirus Disease 2019 (COVID-19)

The present disclosure provides, for example, methods of treating or preventing or delaying progression of COVID-19.

The present disclosure also provides, for example, methods of treating or preventing or delaying progression of a SARS-CoV-2 infection. In some examples of the present disclosure the subject has a SARS-CoV-2 infection but does not have clinically diagnosed COVID-19.

COVID-19 is an infectious disease caused by SARS-CoV-2. Common symptoms include fever, cough, fatigue, shortness of breath, and loss of smell and taste. While the majority of cases result in mild symptoms, some progress to ARDS. The time from exposure to onset of symptoms is typically around five days, but may range from two to fourteen days. There are currently no vaccines nor specific antiviral treatments for COVID-19 and management involves the treatment of symptoms, supportive care, isolation, and experimental measures.

Thus, in some examples, the subject has a SARS-CoV-2 infection. In one example, the subject has COVID-19, for example, severe COVID-19. In particular, severe COVID-19 often results in ARDS. The methods of the present disclosure can be used to treat or prevent or delay progression of ARDS in a subject suffering from severe COVID-19.

### Acute Respiratory Distress Syndrome (ARDS)

The present disclosure provides, for example, methods of treating or preventing or delaying progression of ARDS in a subject.

ARDS is a life-threatening condition characterized by bilateral pulmonary infiltrates, severe hypoxemia, and disruption of the alveolar-capillary membrane barrier (i.e., pulmonary vascular leak), leading to non-cardiogenic pulmonary edema. There is currently no effective pharmacological therapy.

Infectious etiologies, including influenza and coronavirus infection, are leading causes of ARDS. Accordingly, in one example of the present disclosure, the ARDS is associated with a coronavirus infection. For example, a SARS-COV infection. In one example, the ARDS is associated with a SARS-CoV-2 infection.

ARDS is classified according to the Berlin Definition, which includes:
(1) presentation within 1 week of clinical insult or onset of respiratory symptoms;
(2) acute hypoxemic respiratory failure, as determined by a PaO2/FiO2 ratio of 300 mmHg or less on at least 5 cm of continuous positive airway pressure (CPAP) or positive end expiratory pressure (PEEP), where PaO2 is the partial pressure of oxygen in arterial blood and the FiO2 is the fraction of inspired oxygen;
(3) bilateral opacities on lung radiographs not fully explained by effusions, consolidation, or atelectasis; and
(4) edema/respiratory failure not fully explained by cardiac failure or fluid overload.

In one example, the subject has or suffers from ARDS (i.e., the subject satisfies the Berlin definition of ARDS). For example, the subject is in need of treatment (i.e., in need thereof).

In one example, the subject has or suffers from a symptom associated with ARDS. Symptoms associated with ARDS and methods of identifying subjects at risk of developing ARDS will be apparent to the skilled person and/or are described herein. For example, the subject has one or more or all of the following symptoms:
a) a respiratory frequency of greater than 30 breaths per minute;
b) an oxygen saturation (SpO₂) of 93% or less on room air;
c) a ratio of arterial partial pressure of oxygen to fraction of inspired oxygen (PaO₂/FiO₂) of less than 300 mmHg;
d) a SpO₂/FiO₂ ratio of less than 218; and
e) radiographic lung infiltrates in an amount of greater than 50%.

Currently, ARDS is classified as mild, moderate or severe with an associated increased mortality. The severity of ARDS can be categorized according to the Berlin definition as follows:
(i) Mild ARDS: PaO₂/FiO₂ of 200-300 mmHg on at least 5 cm CPAP or PEEP;
(ii) Moderate ARDS: PaO₂/FiO₂ of 100-200 mmHg on at least 5 cm PEEP; and
(iii)Severe ARDS: PaO₂/FiO₂ of less than or equal to 100 mmHg on at least 5 cm PEEP.

In one example, the ARDS is mild ARDS. In another example, the ARDS is moderate ARDS. In a further example, the ARDS is severe ARDS.

The methods of the present disclosure can, in addition to treatment of existing ARDS, be used to prevent the onset of ARDS. Thus, in one example, the subject does not have ARDS.

### Kits

Another example of the disclosure provides kits containing a self-replicating RNA of the present disclosure useful for the treatment or prevention or delaying progression of a disease or disorder as described above.

In one example, the kit comprises (a) a container comprising a self-replicating RNA optionally in a delivery system and/or a pharmaceutically acceptable carrier or diluent; and (b) a package insert with instructions for treating or preventing or delaying progression of a disease or disorder (e.g., COVID-19 or ARDS) in a subject.

In accordance with this example of the disclosure, the package insert is on or associated with the container. Suitable containers include, for example, bottles, vials, syringes, etc. The containers may be formed from a variety of materials such as glass or plastic. The container holds or contains a composition that is effective for a disease or disorder of the disclosure and may have a sterile access port (for example, the container may be an intravenous solution bag or a vial having a stopper pierceable by a hypodermic injection needle). At least one active agent in the composition is the self-replicating RNA. The label or package insert indicates that the composition is used for treating a subject eligible for treatment, e.g., one having or predisposed to developing influenza, an influenza virus infection, a SARS-CoV-2 infection, COVID-19 and/or ARDS, with specific guidance regarding dosing amounts and intervals of treatment and any other medicament being provided. The kit may further comprise an additional container comprising a pharmaceutically acceptable diluent buffer, such as bacteriostatic water for injection (BWFI), phosphate-buffered saline, Ringer's solution, and/or dextrose solution. The kit may further include other materials desirable from a commercial and user standpoint, including other buffers, diluents, filters, needles, and syringes.

The present disclosure includes the following non-limiting Examples.

### EXAMPLES

### Example 1: Generation of the self-replicating RNA

DNA templates encoding the self-replicating RNAs were produced in competent *Escherichia coli* cells that were transformed with a DNA plasmid. Individual bacterial colonies were isolated and the resultant plasmid DNA amplified in *E. coli* cultures. Following fermentation, the plasmid DNA was isolated using Maxiprep DNA kit and linearized by restriction digest. Restriction enzymes were then removed using phenol/chloroform extraction and ethanol precipitation.

mRNA was made by *in vitro* transcription from the linearized DNA template using a T7 RNA polymerase. Subsequently, the DNA template was removed by DNase digestion. Enzymatic capping was performed with Cap0 to provide functional mRNA. The resultant mRNA was purified and resuspended in nuclease-free water.

Self-replicating RNAs were prepared using spike (S) and nucleocapsid (N) antigens from SARS-CoV-2 strain 2019-nCoV/USA-WA1/2020. The following constructs were prepared:
- NSP1-4.SGP.S (wt) (Co5)
- NSP1-4.SGP.N (wt) (Co6)
- NSP1-4.SGP.S (RRAR-QQAA) (Col6)
- NSP1-4.SGP.S (RRAR-QQAA and 986P/987P) (Col7)
- NSP1-4.SGP.S (D614G) (Co48)
- NSP1-4.SGP.S (RRAR-QQAA and D614G) (Co49)
- NSP1-4.SGP.S (RRAR-QQAA and S2') (Co58)
- NSP1-4.SGP.S (RRAR-QQAA, S2' and D614G) (Co59)

### Example 2: In vitro characterisation of the self-replicating RNA

The self-replicating RNAs produced in Example 1 were assessed for expression of the genes of interest.

Two-fold serial dilutions of unformulated (naked) or LNP-formulated self-amplifying mRNA constructs were either electroporated or transfected into a Baby Hamster Kidney (BHK) cell line. After 17-19 hrs, cells were harvested and stained for either S or N antigen expression using anti-S or anti-N antibodies. The number of cells positive for antigen expression and the mean fluorescence intensities (MFIs) were measured by FACS. Data were analysed to calculate the specific potency values (the probability of successful transfection per unit of mass of RNA).

*In vitro* activity and potency of unformulated RNA and LNPs was determined by FACs based on S and N expression and is shown in **Table 1** below:

**Table 1: In vitro activity and potency for unformulated RNA and LNPs**

| **Vector** | **FACS Potency (ng⁻¹)** | **Encapsulation Efficiency (%)** | **SAM recovery (%)** | **Size (d.nm)** | **PDI** | **Zeta pot. (mV)** | **Conductivity (mS/cm)** | **Conc. (µg/mL)** | **Endotoxin (EU/mL)** |
|---|---|---|---|---|---|---|---|---|---|
| Co5 | 3313 | 99.0 | 40 | 108.9 | 0.231 | 33.5 | 0.0539 | 32.4 | <1 |
| Co6 | 11200 | 99.2 | 43 | 98.0 | 0.139 | 32.5 | 0.0569 | 45.3 | <1 |
| Col6 | 4052 | 98.7 | 48 | 96.1 | 0.191 | 31.5 | 0.0582 | 44.6 | <1 |
| Col7 | 5021 | 98.6 | 40 | 93.4 | 0.101 | 36.4 | 0.0590 | 42.1 | <1 |

### Antibody responses

To assess antibody responses, serum was collected at the end of study (i.e., 42 days after first or 21 days after the last, second vaccine dose) and was tested by microneutralization assay (**Table 2**) and ACE2 binding (**Table 3**)**.**

For all serological assays sera were treated in the same way, with Vibrio cholerae neuraminidase, also known as receptor-destroying enzyme (RDE) (Denka Seiken Co. Ltd., Tokyo, Japan) and diluted to a starting dilution of 1:10 with PBS. Sheep serum to H5N1 virus (FDA/CBER Kensington lot nu. H5-Ag-1115) was used as positive control sera three assays.

### Microneutralization assay

Virus fluorescent focus-based microneutralization (FFA MN) assay was performed using in house developed protocol. RDE treated test mouse samples and positive control sera were heat inactivated, diluted to a starting dilution of 1:40 with PBS, and fourfold serial diluted using the U-Bottom 96 well plate (BD Falcon) in neutralization medium (comprised of minimum essential medium D-MEM (GIBCO), supplemented with 1% BSA (Rockland, BSA-30), 100 U/mL penicillin and 100 ug/mL streptomycin (GIBCO)). Virus was diluted to ~ 1,000 - 1,500 fluorescent focus-forming units (FFU)/well (20,000 - 30,000 FFU/mL) in neutralization medium and added in a 1:1 ratio to diluted serum.

After incubation for 2 h at 37°C, 5% CO₂, plates (Half Area 96 well plate, Corning) containing MDCK 33016-PF cells were inoculated with this mixture and incubated overnight for 16 - 18 h at 37°C with 5% CO₂. MDCK 33016-PF cells had been seeded as 3.0E4/well (3.0E6/plate) at 6-8h earlier in the cell growth medium (comprised of D-MEM, supplemented with 10% HyClone fetal bovine serum - FBS (Gibco), 100 U/mL penicillin and 100 ug/mL streptomycin). Following the overnight incubation and prior to immunostaining, cells were fixed with cold mixture of acetone and methanol.

The virus was visualized using separate 1 h incubations at room temperature of monoclonal antibodies specific to the spike (S) protein and Alexa Fluor 488 Goat AntiMouse IgG (H+L) Ab (Invitrogen cat. no. A11001) diluted in PBS buffer containing 0.05% tween-20 (Sigma) and 2% BSA (Fraction V, Calbiochem, 2960, 1194C175). S protein was quantified by a CTL Immunospot analyzer (Cellular Technology Limited, Shaker Heights, Cleveland, OH), using a fluorescein isothiocyanate (FITC) fluorescence filter set with excitation and emission wavelengths of 482 and 536 nm. Fluorescent foci were enumerated by use of software Immunospot 7.0.12.1 professional analyzer DC, using a custom analysis module. The data were successively logged by this software into an Excel data analysis spreadsheet, then 60% focus reduction endpoint was calculated from the average foci count of virus control wells (for each plate), and 60% focus reduction neutralization titer was calculated by linear interpolation between wells immediately above and below the 60% endpoint (for each sample).

**Table 2: Microneutralization assay**

| **Vector** | **MN titer - GMT (1ug)** | **MN titer - GMT (0.01ug)** |
|---|---|---|
| Co16 | 1372.0 | 197.0 |

### Inhibition of ACE2 binding

Inhibition of ACE2 binding was also assessed. The results are shown in **Table 3.**

**Table 3: Inhibition of ACE2 binding**

| **Vector** | **50% inhibition titer - GMT (1ug)** | **50% inhibition titer - GMT (0.01ug)** |
|---|---|---|
| Co16 | 3919 | 886 |

Inhibition of ACE2 binding was also assessed using a surrogate virus neutralization test (sVNT) which detects neutralising antibodies without the need to use any live virus or cells. Using receptor binding domain (RBD) protein from the viral spike (S) protein and the host cell receptor ACE2, this test is designed to mimic the virus-host interaction by direct protein-protein interaction in an ELISA plate well. The highly specific interaction is then neutralized, i.e., blocked by specific NAbs in animal sera in the same manner as in a conventional VNT.

The results are shown in **Table 3A.**

**Table 3A: Inhibition of ACE2 binding**

| **Vector** | **50% inhibition titer - GMT (1ug)** | **50% inhibition titer - GMT (0.01ug)** |
|---|---|---|
| Co5 | 1,095 | 81 |
| Co6 | 5 | 5 |
| Co16 | 1,328 | 9 |
| Co17 | 2,371 | 11 |

### S and N protein antibodies

Antibodies specific to the N protein were also assessed by ELISA. The results are shown in **Table 4.** Antibodies specific to the S protein were also assessed by ELISA. The results are shown in **Table 5.**

**Table 4: N nrotein antibodies**

| | | |
|---|---|---|
| **Vector** | **IgG titer - GMT (1ug)** | **IgG titer - GMT (0.01ug)** |
| Co16 | 1090 | 1090 |

**Table 5: S protein antibodies**

| **Vector** | **IgG titer - GMT (1ug)** | **IgG titer - GMT (0.01ug)** |
|---|---|---|
| Co5 | 8,411 | 1,390 |
| Co6 | - | 1,090 |
| Co16 | 39,205 | 946 |
| Co17 | 35,455 | 1,554 |

### Pseudovirus neutralization

In addition to the sVNT assay, neutralisation capability was assessed using a pseudovirus neutralization assay. The pseudovirus assay is used to demonstrate ability of the constructs to prevent viral entry into a cell. The results are shown in **Table 6.**

**Table 6: Pseudovirus neutralization**

| **Vector** | **IgG titer - GMT (1ug)** | **IgG titer - GMT (0.01ug)** |
|---|---|---|
| Co5 | 1,666 | 1,395 |
| Co6 | 62 | 57 |
| Co16 | 3,379 | 504 |
| Co17 | 13,893 | 397 |

### Cell-mediated immune responses

The self-replicating RNAs Co5, Co6, Co16 (S(QQAA)) and Co17 (S(QQAA; PP) were assessed for their ability to induce antigen specific T cell responses.

Antigen-specific T cell detection was performed on splenic cultures. Briefly, splenocytes were dissociated in dissociation solution (MACS BSA stock 1:20 with autoMACS rinsing solution) and concentrated at 4E7 cells/ml. Briefly, splenocyte cultures were established in 96 well plates in T cell medium containing RPMI, NEAA, pen/strep and βME) and cultured at 37°C/5% CO₂. Anti-CD28 (clone 37.51; BD Biosciences #553294) and anti-CD107a (clone #1D4B; Biolegend #121618) were added to each well. Cell cultures were either stimulated or unstimulated. To stimulate cultures N pep mix (spanning amino acid residues 1-419 of CoV-2 full length N protein), S pep mix 1 (spanning amino acid residues 1-643 of CoV-2 full length S protein), S pep mix 2 (spanning amino acid residues 633-1273 of CoV-2 full length S protein), CoV-1 S peptide (CYGVSATKL) or CoV-2 S peptide (CYGVSPTKL) were added. Following 2 hours of stimulation, Golgi Plug (with brefeldin A; BD Biosciences #555029) was added to each well. Cells were incubated at 37°C for a total of 6 hours after which the cells were transferred to 4°C and stored overnight.

Antigen-specific T cell responses were determined using flow cytometry. Briefly, Fc block mixture (clone 2.4G2; BD Biosciences #553142) was added to each well, followed by extracellular stain (comprising Brilliant stain buffer plus (BD Biosciences #566385), ICOS BV711 (clone C398.4A; Biolegend #313548), CD44 BUV395 (clone IM7; BD Biosciences #740215), CD3 BV786 (clone 145-2C11; BD Biosciences #564379), CD4 APC-H7 (clone GK1.5, BD Biosciences #560181), CD8 AF700 (clone 53-6.7, BD Biosciences #557959) and staining buffer). Cells were stained with UltraComp eBeads (eBiosciences #01-222-42) according to the manufacturer's protocol and incubated at 4°C for 30mins, protected from the light. Cells were washed with staining buffer, centrifuged, resuspended in staining buffer and data acquired using a flow cytometer.

Antigen specific CD4 and CD8 T cell responses were observed to both the N and S constructs. CD4 T cells elicited by sa-mRNA vaccine were mostly Th0 (IL2+ and/or TNFa+, IFNg-, IL5-, IL13-) and Th1 (IFNg+, IL5-, IL13-) with few or no Th2 (IL5+ and/or IL13+, IFNg-) **(****Figure 1****).** Similar frequencies of S1- and S2-reactive CD4 T cells were found; however, for CD8 T cells, S1-reactive T cells dominated over S2-reactive T cells with broad cytokine phenotype, triple, double and single cytokine producing CD8+ T cells.

### IgG subclass

To characterize the type of immune response generated, i.e. Th1 vs Th2 type responses, the S specific IgG1 and IgG2a IgG subclasses were evaluated by ELISA. Little difference between IgG1 and IgG2a response was observed **(Table 7).**

**Table 7: IgG subclasses**

| **Vector** | **IgG1 ELISA GMT (1ug)** | **IgG2a ELISA GMT (1ug)** |
|---|---|---|
| Co16 | 60,367 | 106,625 |

### Example 5: Protective effect of immunization with self-replicating RNAs

To evaluate the protective effect of immunization, hamsters were immunized with Co16 at doses of 3 µg RNA/hamster or 0.3 µg RNA/hamster at Day 1 and Day 22. All animals were challenged 28 days post the second immunization with SARS-CoV-2 US virus intranasally and sacrificed 4 days later, when lung and nasal turbinates were collected for infectious virus measured in lungs and nasal turbinates.

In hamsters, 3.0 and 0.3 µg doses raised neutralization titer GMT as 394 and 270 respectively.

To evaluate protection of lungs from virus infection, average virus recovery from lungs were compared for hamsters immunized with Co16 and control hamsters immunized by PBS. While the viral titer from control hamsters was 5,011,872 TCID50/gr., the average virus recovery from vaccine-immunized hamster was under the limit of quantitation of the assay, <20 TCID50/gr. demonstrating the full protection of lower respiratory track with all vaccines included in the study.

To evaluate the protection of upper respiratory track, virus recovery from the nasal turbinates was measured with the average virus recovery from control hamsters of 120,226,443 TCID50/gr. The viral titer was reduced 10⁴ to 10⁵ folds to 1,995 and 9,120 TCID50/gr. for hamsters immunized with Co16 at doses of 3.0 and 0.3 µg respectively. These results demonstrated that sa-mRNA S significantly reduced viral infection in upper respiratory track.

### Example 6: Double dosing with self-replicating RNAs to SARS-CoV-2

SARS-CoV-2 S and N antigens are not immunologically cross-reactive. To evaluate the antibody immune response in preclinical animal model, female BALB/c mice were immunized at Day 0 with a dose of 1 µg, with a second dose at Day 21. Animals were sacrificed at Day 42 and serum obtained to test for neutralizing antibodies, as well as antibodies inhbiting the binding of S protein to the ACE2 receptor.

The following 1^{st}-2^{nd} dose combinations were assessed:
- PBS-Co6 (N)
- PBS-Co16 (S; RRAR→QQAA)
- Co6-Co6
- Co6-Co16
- Co6-PBS
- Co16-Co16
- Co16-Co6
- Co16-PBS

### S and N protein antibodies

Antibodies specific to the S and N proteins were assessed by ELISA at Day 42. The results are shown in **Table 8.**

**Table 8: S protein antibodies following prime-boost**

| **Prime** | **Boost** | **IgG titer (GMT) to S protein** | **IgG titer (GMT) to N protein** |
|---|---|---|---|
| PBS | Co6 | ND | 586882 |
| PBS | Co16 | 44834 | 500 |
| Co6 | Co6 | 500 | 3118749 |
| Co6 | Co16 | 73696 | 2404559 |
| Co6 | PBS | 500 | 611827 |
| Co16 | Co16 | 2117875 | 500 |
| Co16 | Co6 | 24562 | 625381 |
| Co16 | PBS | 67148 | 789 |

Homologous prime/boost was more effective than heterologous prime-boost with regards an anti-S response, however heterologous prime-boost was more effective than homologous prime-boost with regards an anti-N response. In addition, boost with S (i.e., Co16) increases anti-N responses compared to boost with PBS.

In the absence of a boost, anti-S antibodies increased from day 21 to day 42 (data not shown).

### Inhibition of ACE2 binding

Inhibition of ACE2 binding was also assessed. The results are shown in **Table 9.**

**Table 9: Inhibition of ACE2 binding**

| **Prime** | **Boost** | **50% inhibition of ACE2 binding (GMT)** |
|---|---|---|
| PBS | Co6 | ND |
| PBS | Co16 | 594 |
| Co6 | Co6 | 15 |
| Co6 | Co16 | 1307 |
| Co6 | PBS | 15 |
| Co16 | Co16 | 31065 |
| Co16 | Co6 | 349 |
| Co16 | PBS | 1312 |

### Microneutralization assay

WT virus neutralization was also assessed. The results are shown in **Table 10.**

**Table 10: WT virus neutralisation**

| **Prime** | **Boost** | **MN titer (GMT)** |
|---|---|---|
| PBS | Co6 | 5 |
| PBS | Co16 | 92 |
| Co6 | Co6 | 5 |
| Co6 | Co16 | 98 |
| Co6 | PBS | 5 |
| Co16 | Co16 | 2744 |
| Co16 | Co6 | 46 |
| Co16 | PBS | 211 |

### Cell-mediated immune responses

Antigen specific T cell responses were also assessed. CD4 and CD8 T cell responses were observed following vaccination with both homologous and heterologous antigens.

### Example 6: Generation of self-replicating RNA variants

Self-replicating RNAs were prepared using spike (S) antigens from SARS-CoV-2 variant strains, namely. the UK alpha strain (B.1.1.7), the South African beta (B.1.351) strains. The following constructs were prepared:
- NSP1-4.SGP.S (RRAR-QQAA) (Co16)
- NSP1-4.SGP.S (RRAR-QQAA and D614G) (Co49)
- NSP1-4.SGP.S (RRAR-QQAA, S2' (R815N) and D614G) (Co59)
- NSP1-4.SGP.S(RRAR→QQAA; Δ69-70; ΔY144; N501Y; D614G) (Co77)
- NSP1-4.SGP.S(RRAR→QQAA; Δ242-244; K417N; E484K; N501Y; D614G) (Co78)
- NSP1-4.SGP.S(RRAR→QQAA; Δ69-70; Δ242-244; K417N; E484K; N501Y; D614G) (Co79)
- NSP1-4.SGP.S(RRAR→QQAA; Δ69-70; ΔY144; N501Y; A570D; D614G; P680H; T716I) (Co80)
- NSP1-4.SGP.S(RRAR→QQAA; L18F; D80A; D215G; Δ242-244; K417N; E484K; N501Y; D614G; A701V) (Co81)

*In vitro* activity and potency of LNP formulated RNA was determined as described above and is shown in **Table 11** below:

**Table 11: In vitro potency for formulated RNA in LNPs**

| **Vector** | **FACS Potency (ng⁻¹)** | **Encapsulation Efficiency (%)** | **SAM recovery (%)** | **Size (d.nm)** | **PDI** | **Zeta pot. (mV)** | **Conductivity (mS/cm)** | **Conc. (µg/mL)** | **Endotoxin (EU/mL)** |
|---|---|---|---|---|---|---|---|---|---|
| Col6 | 2845 | 95.9 | 38.8 | 98.5 | 0.179 | 32.6 | 0.0559 | 35.8 | <1 |
| Co77 | 3722 | 96.9 | 31.0 | 100.8 | 0.156 | 40.6 | 0.0546 | 31.0 | <1 |
| Co78 | 2448 | 97.8 | 50.3 | 96.2 | 0.152 | 38.2 | 0.0574 | 48.9 | <1 |

As shown in **Table 12,** all constructs have *in vitro* activity and potency

**Table 12: In vitro potency and activity**

| **Vector** | **RNA activity** | **LNP potency** |
|---|---|---|
| Co16 | 142 | 2845 |
| Co49 | 172 | 3099 |
| Co59 | 118 | 1771 |
| Co77 | 224 | 3722 |
| Co78 | 202 | 2448 |
| Co79 | 260 | 3008 |
| Co80 | 175 | 1918 |
| Co81 | 171 | 2309 |

### Neutralisation assay

To determine the neutralisation capabilities of the constructs, a microneutralization assay was performed against the reference Whuan sequence, as well as the alpha variant (B.1.1.7; UK strain); beta variant (B.1.351; South Aftrican strain); gamma variant (P.1; Brazillian strain); and delta variant (B.1.617.2; Indian strain).

As shown in **Figure 2****,** all constructs generated an immune response against all strains.

### Inhibition of ACE2 binding

Inhibition of ACE2 binding was also assessed. The results are shown in **Table 13.** All constructs inhibited ACE2 binding at Day 42.

**Table 13: Inhibition of ACE2 binding**

| **Vector** | **Log₁₀ inhibition of ACE2 binding (GMT) 1.00µg** | **Log₁₀ inhibition of ACE2 binding (GMT) 0.01µg** |
|---|---|---|
| | **Original target strain** | |
| Co16 | 8437 | 1646 |
| Co77 | 7269 | 1062 |
| Co78 | 1479 | 192 |

| | **Alpha target strain** | |
|---|---|---|
| Co16 | 7119 | 854 |
| Co77 | 8351 | 1171 |
| Co78 | 848 | 130 |

| | **Beta target strain** | |
|---|---|---|
| Co16 | 3159 | 247 |
| Co77 | 4113 | 446 |
| Co78 | 3560 | 725 |

| | **Delta target strain** | |
|---|---|---|
| Co16 | 1379 | 336 |
| Co77 | 733 | 83 |
| Co78 | 34 | 19 |

### IgG ELISA

As shown in **Figure 3****,** all constructs generated total Ig responses at high and low doses. All constructs generated responses that are highly cross-reactive.

### Cell-mediated immune responses

The frequency of B cells induced by the variant constructs was characterised.

As shown in **Figure 4** and **Table 14,** all constructs generated S-specific B cells that reacted with all variant B-cell receptor specific probes. Non-specific controls (i.e., no bait and negative control HA H1) showed low levels of background binding.

Antigen-specific T cell responses were determined using flow cytometry as described above. Peptide pools (as described above) matched the original CoV-2 strain and not the variant strains. All constructs induced antigen-specific CD4 and CD8 T cells reactive with S1 and S2 epitopes **(****Figure 5** and **Table 14).** CD4 T cells were mostly Th0 (IL2+ and/or TNFa+, IFNg-, IL5-, IL13-) and Th1 (IFNg+, IL5-, IL13-) with few or no Th2 (IL5+ and/or IL13+, IFNg-).

**Table 15: Cell-mediated immune responses**

| | **B cells** | **T cells** | |
|---|---|---|---|
| | | **CD4** | **CD8** |
| Co16 | 1.00 | 1.00 | 1.00 |
| Co77 | 0.70 | 0.81 | 0.76 |
| Co80 | 0.66 | 0.94 | 0.96 |
| Co78 | 0.54 | 1.07 | 1.41 |
| Co81 | 0.86 | 0.85 | 1.13 |
| Co79 | 0.76 | 0.69 | 1.20 |
| Co49 | 0.84 | 0.99 | 0.98 |
| Co59 | 0.47 | 0.76 | 1.28 |

Disclosed herein are the following embodiments:
Embodiment 1. A self-replicating RNA comprising a nucleotide sequence encoding an antigen operably linked to a subgenomic promoter, wherein the antigen is from a severe acute respiratory syndrome coronavirus 2 (SARS-CoV-2).
Embodiment 2. A monocistronic self-replicating RNA comprising a nucleotide sequence encoding an antigen operably linked to a subgenomic promoter, wherein the antigen is from a SARS-CoV-2.
Embodiment 3. The self-replicating RNA of claim 1 or 2, wherein the antigen is a spike (S) protein or a nucleocapsid (N) protein.
Embodiment 4. The self-replicating RNA of claim 3, wherein the S protein is encoded by a sequence set forth in SEQ ID NO: 1.
Embodiment 5. The self-replicating RNA of claim 3, wherein the S protein is a mutant S protein.
Embodiment 6. The self-replicating RNA of claim 5, wherein the mutant S protein:
   (i) lacks a furin cleavage site at the S1/S2 boundary and comprises RRAR to QQAA mutations at residues corresponding to nucleotides 682-685 of SEQ ID NO: 18; and/or
   (ii) lacks a furin cleavage site at the S2' site; and/or
   (iii) comprises D to G mutation at residue corresponding to nucleotide 614 of SEQ ID NO: 18; and/or
   (iv) comprises insertion of two proline residues between residues corresponding to nucleotides 986 and 987 of SEQ ID NO: 18.
Embodiment 7. The self-replicating RNA of claims 5 or 6, wherein the mutant S protein is encoded by a sequence set forth in any one of SEQ ID NOs: 2 to 7 and/or SEQ ID NOs: 19-23.
Embodiment 8. The self-replicating RNA of claim 3, wherein the N protein is encoded by a sequence set forth in SEQ ID NO: 8.
Embodiment 9. The self-replicating RNA of any one of claims 1 to 8, wherein the SG promoter is encoded by a sequence set forth in SEQ ID NO: 9.
Embodiment 10. The self-replicating RNA of any one of claims 1 to 9, wherein the self-replicating RNA is from an alphavirus.
Embodiment 11. The self-replicating RNA of claim 10, wherein the alphavirus is selected from the group consisting of Semliki Forest virus (SFV), Sindbis virus (SIN), and Venezuelan equine encephalitis virus (VEEV) and combinations thereof.
Embodiment 12. The self-replicating RNA of any one of claims 1 to 11, wherein the RNA is encoded by a sequence set forth in any one of SEQ ID NOs: 10 to 17.
Embodiment 13. An immunogenic composition comprising the self-replicating RNA of any one of claims 1 to 12.
Embodiment 14. The immunogenic composition of claim 13, comprising a plurality of self-replicating RNAs of any one of claims 1 to 12, wherein each self-replicating RNA encodes a different polypeptide antigen sequence.
Embodiment 15. A pharmaceutical composition comprising an immunogenic composition of claims 13 or 14 and a pharmaceutically acceptable carrier. Embodiment 16. The pharmaceutical composition of claim 15, further comprising a lipid nanoparticle (LNP), a polymeric microparticle or an oil-in-water emulsion.
Embodiment 17. The pharmaceutical composition of claim 16, wherein the self-replicating RNA is encapsulated in, bound to or adsorbed on a LNP, a polymeric microparticle or an oil-in-water emulsion.
Embodiment 18. The immunogenic composition of claims 13 or 14 or the pharmaceutical composition of any one of claims 15 to 17 for use as a vaccine.
Embodiment 19. The immunogenic composition of claims 13 or 14, or the pharmaceutical composition of any one of claims 15 to 17 for use in the treatment or prevention or delaying progression of a disease or condition selected from the group consisting of SARS-CoV-2 infection, coronavirus disease 2019 (COVID-19), acute respiratory disease syndrome (ARDS) and combinations thereof.
Embodiment 20. A method of treating or preventing or delaying progression of a disease or condition in a subject, the method comprising administering the immunogenic composition of claims 13 or 14, or the pharmaceutical composition of any one of claims 15 to 17 to a subject in need thereof.
Embodiment 21. Use of the self-replicating RNA of any one of claims 1 to 12, or the immunogenic composition of claims 13 or 14, or the pharmaceutical composition of any one of claims 15 to 17 in the manufacture of a medicament for treating or preventing or delaying progression of a disease or condition in a subject in need thereof.
Embodiment 22. The method of claim 20, or the use of claim 21, wherein the disease or condition is selected from the group consisting of a SARS-CoV-2 infection, COVID-19, ARDS and combinations thereof.
Embodiment 23. A method of inducing an immune response in a subject, the method comprising administering the immunogenic composition of claims 13 or 14, or the pharmaceutical composition of any one of claims 15 to 17 to a subject in need thereof.
Embodiment 24. The method of claim 23, wherein the immune response is a humoral and/or a cell-mediated immune response.
Embodiment 25. Use of the self-replicating RNA of any one of claims 1 to 12, or the immunogenic composition of claims 13 or 14, or the pharmaceutical composition of any one of claims 15 to 17 in the manufacture of a medicament for inducing an immune response in a subject in need thereof.
Embodiment 26. A polynucleotide encoding the self-replicating RNA of any one of claims 1 to 12.
Embodiment 27. The polynucleotide of claim 26, wherein the polynucleotide is a recombinant DNA.
Embodiment 28. The polynucleotide of claim 27, wherein the recombinant DNA is a plasmid.
Embodiment 29. The polynucleotide of claim 28, wherein the plasmid comprises a sequence set forth in any one of SEQ ID NOs: 10 to 17.

## Claims

1. A self-replicating RNA comprising a nucleotide sequence encoding an antigen operably linked to a subgenomic promoter, wherein the antigen is from a severe acute respiratory syndrome coronavirus 2 (SARS-CoV-2), wherein the antigen is a spike (S) protein, wherein the S protein is a mutant S protein and wherein the mutant S protein:
(i) comprises D to G mutation at residue corresponding to nucleotide 614 of SEQ ID NO: 18; and
(ii) comprises insertion of two proline residues between residues corresponding to nucleotides 986 and 987 of SEQ ID NO: 18.

2. The self-replicating RNA of claim 1, wherein the self-replicating RNA further comprises a Kozak consensus sequence set forth in SEQ ID NO: 36.

3. The self-replicating RNA of claims 1 or 2, wherein the SG promoter comprises a sequence set forth in SEQ ID NO: 9.

4. The self-replicating RNA of any one of claims 1 to 3, wherein the self-replicating RNA is from an alphavirus, for example, the alphavirus is selected from the group consisting of Venezuelan equine encephalitis virus (VEEV), Semliki Forest virus (SFV), Sindbis virus (SIN), and combinations thereof.

5. The self-replicating RNA of claim 4, wherein the self-replicating RNA comprises a 5' untranslated region of a Venezuelan equine encephalitis virus (VEEV).

6. The self-replicating RNA of any one of claims 1 to 5, wherein the mutant S protein lacks a furin cleavage site at the S2' site.

7. An immunogenic composition comprising the self-replicating RNA of any one of claims 1 to 6, for example, comprising a plurality of self-replicating RNAs of any one of claims 1 to 6.

8. A pharmaceutical composition comprising an immunogenic composition of claim 7 and a pharmaceutically acceptable carrier.

9. The pharmaceutical composition of claim 8, further comprising a lipid nanoparticle (LNP), a polymeric microparticle or an oil-in-water emulsion, for example, the self-replicating RNA is encapsulated in, bound to or adsorbed on a LNP, a polymeric microparticle or an oil-in-water emulsion.

10. The immunogenic composition of claim 6 or the pharmaceutical composition of claims 8 to 9, for use as a vaccine or for use in the treatment or prevention or delaying progression of a disease or condition selected from the group consisting of SARS-CoV-2 infection, coronavirus disease 2019 (COVID-19), acute respiratory disease syndrome (ARDS) and combinations thereof, or for inducing an immune response in a subject, wherein the immune response is a humoral and/or a cell-mediated immune response.

11. A polynucleotide encoding the self-replicating RNA of any one of claims 1 to 6, for example, the polynucleotide is a recombinant DNA such as a plasmid.
